(19) Europäisches Patentamt
European Patent Office
Office européen des brevets

(11) **EP 1 959 816 B1**

(12) **EUROPÄISCHE PATENTSCHRIFT**

(45) Veröffentlichungstag und Bekanntmachung des Hinweises auf die Patenterteilung:
**20.07.2011 Patentblatt 2011/29**

(51) Int Cl.:
***A61B 3/10*** *(2006.01)*    ***G01B 9/02*** *(2006.01)*

(21) Anmeldenummer: **06829365.3**

(22) Anmeldetag: **06.12.2006**

(86) Internationale Anmeldenummer:
**PCT/EP2006/011738**

(87) Internationale Veröffentlichungsnummer:
**WO 2007/065670 (14.06.2007 Gazette 2007/24)**

(54) **INTERFEROMETRISCHE PROBENMESSUNG**

INTERFEROMETRIC SAMPLE MEASUREMENT

MESURE INTERFEROMETRIQUE D'UN ORGANE

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **06.12.2005 DE 102005058220**
**16.08.2006 AT 13742006**

(43) Veröffentlichungstag der Anmeldung:
**27.08.2008 Patentblatt 2008/35**

(73) Patentinhaber: **Carl Zeiss Meditec AG**
**07745 Jena (DE)**

(72) Erfinder:
• **FERCHER, Adolf, Friedrich**
**A-1230 Wien (AT)**
• **LEITGEB, Rainer**
**A-1030 Wien (AT)**

(74) Vertreter: **Geyer, Fehners & Partner**
**Patentanwälte**
**Perhamerstrasse 31**
**80687 München (DE)**

(56) Entgegenhaltungen:
WO-A-01/19303    WO-A-01/38820
WO-A-03/062802    WO-A-2006/015717
WO-A2-03/086180    AT-A1- 500 501
US-B1- 6 256 102

• **FERCHER A F: "OPTICAL COHERENCE TOMOGRAPHY" JOURNAL OF BIOMEDICAL OPTICS, INTERNATIONAL SOCIETY FOR OPTICAL ENGINEERING (SPIE), US, Bd. 1, Nr. 2, April 1996 (1996-04), Seiten 157-173, XP000863155 ISSN: 1083-3668**
• **FERCHER A F ET AL: "Optical coherence tomography-principles and applications" REPORTS ON PROGRESS IN PHYSICS, INSTITUTE OF PHYSICS PUBLISHING, BRISTOL, GB, Bd. 66, 2003, Seiten 239-303, XP002346303 ISSN: 0034-4885**
• **WOJTKOWSKI M ET AL: "In vivo human retinal imaging by Fourier domain optical coherence tomography" JOURNAL OF BIOMEDICAL OPTICS, SPIE, BELLINGHAM, WA, US, Bd. 7, Nr. 3, Juli 2002 (2002-07), Seiten 457-463, XP002272406 ISSN: 1083-3668**

EP 1 959 816 B1

**Beschreibung**

Die Erfindung bezieht sich auf eine Vorrichtung zur interferometrischen Messung einer Probe, insbesondere des Auges, mit einer Kurzkohärenz-Interferometeranordnung, welche einen Meßstrahlengang, durch den ein Meßstrahl auf die Probe fällt, und einen Referenzstrahlengang aufweist, durch den ein Referenzstrahl läuft, der mit dem Meßstrahl überlagert und zur Interferenz gebracht wird. Die Erfindung bezieht sich weiter auf ein Verfahren zur kurzkohärenz-interferometrischen Messung einer Probe, insbesondere des Auges, wobei durch einen ersten Meßstrahlengang ein Meßstrahl auf die Probe gerichtet wird und mit einem Referenzstrahl, der einen Referenzstrahlengang durchläuft, überlagert und zur Interferenz gebracht wird.

Zur interferometrischen Messung oder Vermessung von Proben ist die optische Kurzkohärenztomographie (auch OCT) bekannt. Mit diesem Prinzip lassen sich mit hoher Empfindlichkeit optische Schnitte im Material vermessen, wobei axiale Auflösungen, d. h. Auflösungen entlang der optischen Einfallsachse der Strahlung, von wenigen Mikrometern erreicht werden. Das Prinzip basiert auf der optischen Interferometrie und verwendet für eine Auflösung in der Tiefenrichtung, d. h. entlang der optischen Achse, eine teilkohärente Lichtquelle.

Eine bekannte Anwendung für die optische Kurzkohärenztomographie ist die Vermessung des Auges, insbesondere des menschlichen Auges. Die Carl Zeiss Meditec AG vertreibt dazu unter der Bezeichnung IOL-Master ein Gerät, das unter anderem die Augenlänge, d. h. den Abstand zwischen Corneascheitel und Fundus, bestimmt. Hierbei wird während der Messung die Weglänge des Referenzstrahls verändert. Anwendung findet das Gerät insbesondere im Rahmen der Katarakt-Chirurgie. Die Brechkraft einer zu implantierenden Intraokularlinse wird bei der Katarakt-Chirurgie und der refraktiven Augenchirurgie aus dem refraktiven Ausgangszustands des Auges, der akustisch oder optisch bestimmten Länge des Auges und einer Abschätzung der postoperativen Vorderkammertiefe bestimmt. Man benötigt also vor dem Eingriff genaue Kenntnis über diese Parameter. Der Scanvorgang des IOL-Masters liefert am Interferometerausgang ein Signal, aus dessen zeitlichem Ablauf die zu messenden Längen bestimmt werden. Dieser Scanvorgang braucht Zeit; Bewegungen des Probanden während der Messung haben Fehler oder ungenaue Ergebnisse zur Folge.

Aus der WO03/086180 ist eine kunzkohärenz- luter ferometer anordnung mit einem Referenzarm mit mehreren Strahlerflexionen bekannt.

Weiter ist eine fourieranalysierende Interferenzmethode bekannt. Zur Ortsauflösung in Tiefenrichtung zeichnet man ein Spektrum des Interferenzmusters zwischen Referenzstrahl und Meßstrahl auf, der an der Probe rückgestreut wurde. Diese Aufzeichnung kann simultan mittels eines Spektrometers (bei geeigneter Breitbandlichtquelle) oder sequentiell (bei durchstimmbaren Quellen) erfolgen. Die inverse Fouriertransformierte des Spektrums ermöglicht eine Rekonstruktion der Struktur entlang der Tiefenrichtung.

Der Erfindung liegt deshalb die Aufgabe zugrunde, eine Vorrichtung der eingangs genannten Art so fortzubilden, daß größere Teilstrecken des Auges schnell gemessen werden können.

Diese Aufgabe löst die Erfindung dadurch, daß die Interferometeranordnung für unterschiedliche, axial beabstandete Probenbereiche hinsichtlich der Längen der Referenzstrahlengänge vorabgestimmt ist. Referenzstrahlengänge sind räumlich getrennt und unterschiedlich lang, wobei die Weglängendifferenz einen Abstand der Meßbereiche in der Probe vorgibt. Die OCT-Messung mißt dann nur die Abweichung von voreingestelltem Abstand. Die Strahlung aus dem jeweiligen Referenzstrahlengang wird mit dem Meßstrahl eigenständig überlagert und zur Detektion gebracht.

Die Erfindung nutzt also z. B. die Fourier-Domain Kurzkohärenz-Interferometrie (FD OCT), welche das Wellenzahl-Spektrum des Signals am Interferometerausgang verwertet. Dieses Spektrum wird z. B. mittels eines Spektrometers aufgenommen, welches üblicherweise ein dispergierendes Element, beispielsweise ein Beugungsgitter, und eine fokussierende Optik sowie ein Detektor-Array, z. B. Photodioden-Arrays oder Array-Kameras, enthält. Das mittels des Detektorarrays registrierte Wellenlängenspektrum $I(\lambda.)$ wird beispielsweise mit Hilfe der Gittergleichung in das benötigte Signalspektrum oder K-Spektrum $I(K)$ umgerechnet. Der von typischen Photodioden-Arrays oder Array-Kameras elektronisch ausgeführte Scan ist sehr schnell, er benötigt wenige Millisekunden oder Bruchteile einer Millisekunde. Gleiches gilt für das Durchstimmen frequenzveränderlicher Strahlungsquellen, wenn man mit spektral nichtselektiven Detektoren arbeitet. Damit ist die für Messungen an Patienten vorteilhafte "One-Shot"-Qualität, bei der die relevanten Meßdaten aus einer einzigen oder sehr kurzen Belichtung des Auges gewonnen werden, erreicht.

Die Fourier-Transformation des K-Spektrums liefert ein tiefenabhängiges Signal mit Signalspitzen, deren z-Position die Wegdifferenz Referenzstrahl/Meßstrahl angibt.

Die Auflösung $\Delta z$ der Kurzkohärenz-Interferometrie hängt mit der Halbwertsbreite $\Delta\lambda$, des Wellenlängenspektrums und dessen mittlerer Wellenlänge $\overline{\lambda}$ zusammen. Für ein Gauß'sches Spektrum erhält man:

$$\Delta z = \frac{2\ln 2}{\pi} \frac{\overline{\lambda}^2}{\Delta\lambda}.$$

Die Meßfeldtiefe Z ist durch die Pixelzahl oder Anzahl N der Array-Photodioden in der Dispersionsrichtung des Spektrometers bzw. durch die Anzahl an Aufnahmen während des Durchstimmens begrenzt. Hier gilt $N = \Delta K \cdot \dfrac{z}{\pi}$, worin $\Delta K$ die Bandbreite der benutzten optischen Strahlung im K-Raum ist. Die Meßfeldtiefe ist also

$$Z = \frac{N}{4} \frac{\overline{\lambda}^2}{\Delta \lambda}.$$

Sie hängt linear von der Pixelzahl N des Spektrometers bzw. der Aufnahmenanzahl ab. Übliche Array-Pixel-zahlen von $N \approx 1000$ liefern Meßfeldtiefen $Z$ von rund 5,3 mm. Der Ursprung des Meßfelds ist die "Wegdifferenz-Null-Position", das ist jene Position im Meßstrahl, für welche die optische Länge des Meßstrahls gleich der des Referenzstrahl ist.

Die Erfindung kann verwirklicht werden durch eine Vorrichtung zur interferometrischen Messung einer Probe, insbesondere des Auges, mit einer Kurzkohärenz-Interferometeranordnung, welche einen Meßstrahlengang, durch den ein Meßstrahl auf die Probe fällt, und einen ersten Referenzstrahlengang aufweist, durch den ein Referenzstrahl läuft, der mit dem Meßstrahl überlagert und zur Interferenz gebracht wird, wobei die Interferometeranordnung mindestens einen zweiten Referenzstrahlengang aufweist, der vom ersten Referenzstrahlengang zumindest teilweise räumlich getrennt verläuft und dessen optischen Weglänge sich von der des ersten Referenzstrahlenganges unterscheidet, wobei die Weglängendifferenz gemäß einem Abstand zweier in Tiefenrichtung der Probe beabstandeter Probenbereiche gewählt ist und wobei eine Steuereinrichtung aus den detektierten überlagerten Strahlen mittels Fourier-Spektralanalyse unter Berücksichtigung der Weglängendifferenz der Referenzstrahlengänge den Abstand der Probenbereiche ermittelt. Analog kann die Erfindung realisiert werden durch ein Verfahren zur kurzkohärenz-interferometrischen Messung einer Probe, insbesondere des Auges, wobei durch einen Meßstrahlengang ein Meßstrahl auf die Probe gerichtet wird und mit einem Referenzstrahl, der einen ersten Referenzstrahlengang durchläuft, überlagert und zur Interferenz gebracht wird, wobei mindestens ein zweiter Referenzstrahlengang vorgesehen wird, der vom ersten Referenzstrahlengang zumindest teilweise getrennt verläuft und dessen optischen Weglänge sich von der des ersten Referenzstrahlenganges unterscheidet, wobei die Weglängendifferenz gemäß einem Abstand zweier in Tiefenrichtung der Probe beabstandeter Probenbereiche gewählt wird und die überlagerte Strahlung detektiert und daraus mittels Fourier-Spektralanalyse unter Berücksichtigung der Weglängendifferenz der Referenzstrahlengänge der Abstand der Probenbereiche ermittelt wird.

In der Interferometeranordnung ist es für die Signalgüte vorteilhaft, die Strahlung aus den beiden Referenzstrahlengängen jeweils getrennt mit der von der Probe rückreflektierten Meßstrahl zu überlagern und nachzuweisen. Es ist deshalb in einer Weiterbildung vorgesehen, daß die Interferometeranordnung eine Überlagerungseinrichtung aufweist, die die Referenzstrahlen aus den zwei Referenzstrahlengängen getrennt mit dem Meßstrahl aus dem Meßstrahlengang überlagert und die derart überlagerten Strahlen dann an eine Detektoreinrichtung zum Nachweis leitet, welche den beabstandeten Meßbereichen zugeordnete Meßsignale erzeugt. Analog ist für das Verfahren vorgesehen, daß die Strahlen aus den zwei Referenzstrahlengängen getrennt mit dem Meßstrahl aus dem Meßstrahlengang überlagert, die derart überlagerten Strahlen getrennt detektiert und den beabstandeten Meßbereichen zugeordnete Meßsignale erzeugt werden.

Die Trennung bei Überlagerung und Nachweis kann auf verschiedenste Art und Weise erfolgen. Zum einen ist eine zeitliche Trennung möglich. Der Meßstrahl wird also nacheinander mit den Referenzstrahlen aus den Referenzstrahlengängen überlagert und nachgewiesen. Dies hat den Vorteil, daß nur eine Detektoreinheit notwendig ist, auf Kosten einer etwas längeren Meßdauer. Daß nur eine Spektrafanalysevorrichtung detektionsseitig notwendig ist, kann den Aufwand und damit die Kosten erheblich reduzieren. Es ist deshalb in einer Variante der Erfindung vorgesehen, daß die Überlagerungseinrichtung einen Umschaltmechanismus zum Umschalten zwischen den zwei Referenzstrahlengängen aufweist, so daß die Überlagerung für die zwei Referenzstrahlengänge sequentiell erfolgt. Für das Verfahren ist analog vorgesehen, daß zwischen den zwei Referenzstrahlengängen sequentiell umgeschaltet wird, so daß der Meßstrahl sequentiell mit Strahlung aus dem ersten und dem zweiten Referenzstrahlengang überlagert wird und die überlagerten Strahlen sequentiell detektiert werden.

Eine höhere Meßgeschwindigkeit erreicht man zum anderen, wenn die Überlagerung derart ist, daß ein paralleler Nachweis der getrennt überlagerten Strahlen, d. h. der für die unterschiedlichen Meßbereiche erzeugten Interferenzmuster erfolgt. Ein erster Ansatz verwendet eine Polarisationstrennung. So sind beispielsweise zwei Referenzstrahlengänge vorgesehen, die zueinander orthogonal polarisierte Strahlung führen. Detektionsseitig sind dann ebenfalls zwei Detektoreinheiten vorgesehen, die ebenfalls zueinander orthogonale Strahlungsanteile der überlagerten Strahlung auswerten. Es ist für diese erste Variante deshalb zweckmäßig, daß die Überlagerungseinrichtung zur getrennten Überlagerung eine Polarisationstrennung einsetzt, so daß die Überlagerung und das Weiterleiten zur Detektoreinrichtung für die zwei Referenzstrahlengänge nach Polarisation getrennt und gleichzeitig erfolgt. Weiter ist es für das Verfahren zweckmäßig, daß zur getrennten Überlagerung eine Polarisationstrennung einsetzt wird, so daß die getrennte Überlagerung und Detektion für die zwei Referenzstrahlengänge nach Polarisation getrennt und gleichzeitig erfolgt.

Eine weitere Trennmöglichkeit ergibt sich in einer zweiten Variante durch die Verwendung verschiedener Wellenlängenbereiche. Die Referenzstrahlengänge können dann durch dichroitische Aufteilung der einfallenden Referenzstrahlung aneinander gekoppelt werden und einer entsprechenden Anzahl von Detektoreinrichtungen ist ebenfalls eine entsprechende dichroitische Trennung vorgeordnet. Es ist für diese zweite Variante deshalb zweckmäßig, daß die Überlagerungseinrichtung zur getrennten Überlagerung eine dichroitische Trennung einsetzt, so daß die Überlagerung und das Weiterleiten zur Detektoreinrichtung für die zwei Referenzstrahlengänge spektral getrennt und gleichzeitig erfolgt. Analog ist für das Verfahren vorgesehen, daß zur getrennten Überlagerung eine dichroitische Trennung einsetzt wird, so daß die getrennte Überlagerung und Detektion für die zwei Referenzstrahlengänge spektral getrennt und gleichzeitig erfolgt.

Eine weitere Trennung, die eine simultane Messung ermöglicht, liegt in einer räumlichen Trennung der überlagerten Strahlung. In dieser dritten Variante wird die Meßstrahlung räumlich auseinandergeführt und jeweils mit entsprechend räumlich getrennten Referenzstrahlen aus den Referenzstrahlengängen überlagert. Die räumliche Trennung kann insbesondere als Pupillenteilung realisiert sein, weshalb in einer Weiterbildung vorgesehen ist, daß die Überlagerungseinrichtung zur getrennten Überlagerung eine Pupillenteilung einsetzt, so daß die Überlagerung und das Weiterleiten zur Detektoreinrichtung für die zwei Referenzstrahlengänge in einer geteilten Pupille des Strahlenganges erfolgt.

Das erfindungsgemäße Konzept verwendet, wie bereits erwähnt, Kurzkohärenz-FD OCT. Dabei kann das benötigte K-Spektrum sowohl mit spektral sensitiver Detektion und breitbandigen Quellen als auch mit spektral nichtauflösender Detektion und Durchstimmung einer schmalbandigen Quelle erzeugt werden. Natürlich verringert sich der Detektoraufwand, wenn eine die Interferometeranordnung speisende, spektral durchstimmbare Strahlungsquelle und eine spektral nichtauflösende Detektoreinrichtung zur Messung vorgesehen sind. Das K-Spektrum wird hier aus den Daten über die Durchstimmung zusammengesetzt und dann analysiert.

Bei FD OCT bestand bislang eine Problematik, daß man mit den verfügbaren Detektoren mit einer vorgegebenen Maximalanzahl an Pixeln bzw. Aufnahmen pro Zeiteinheit entweder die spektrale Auflösung und damit die Ortsauflösung maximieren konnte, oder den abgedeckten Spektralbereich, mithin den Meßbereich. Das erfindungsgemäße Konzept löst diese gegenläufige Kopplung nun dadurch auf, daß mehrere unterschiedlich lange Referenzstrahlengänge verwendet werden.

Die gemessenen Abstände sind üblicherweise auf den Abstand zum Interferometer bzw. zur bereits eingangs erwähnten Wegdifferenz-Null-Position bezogen. In einer weiteren Erfindung ist es möglich, diese Meßsignale aufeinander zu beziehen. Dies erfolgt dadurch, daß der Referenzstrahlengang nicht mehr die Reflektion an einem statischen, im Interferometer eingebauten Reflektor vornimmt, sondern einen auf die Probe einfallenden und von dort rückreflektierten oder -gestreuten Strahl als Referenzstrahl verwendet. Es ist für die eingangs genannte Interferometeranordnung deshalb erfindungsgemäß auch vorgesehen, daß der Referenzstrahlengang die Probe umfaßt, wobei Referenzstrahl und Meßstrahl gegeneinander in Strahlrichtung um eine bestimmte Weglängendifferenz versetzt sind und der Referenzstrahl an einem ersten Probenbereich und der Meßstrahl an einem zweiten Probenbereich der Probe reflektiert und/oder rückgestreut sind und die Interferenz zwischen Meßstrahl und Referenzstrahl vom Abstand zwischen den beiden Probenbereichen abhängt, wobei eine Steuereinrichtung aus den detektierten überlagerten Strahlen mittels Fourier-Spektralanalyse unter Berücksichtigung der Weglängendifferenz der Refererizstrahlengänge den Abstand der Probenbereiche ermittelt. Analog ist für das Verfahren vorgesehen, daß der Referenzstrahlengang umfaßt, Referenzstrahl und Meßstrahl gegeneinander in Strahlrichtung um eine bestimmte Weglängendifferenz versetzt sind und, der Referenzstrahl an einem ersten Probenbereich und der Meßstrahl an einem zweiten Probenbereich der Probe reflektiert und/oder rückgestreut werden und der Abstand zwischen den beiden Probenbereichen aus der Interferenz zwischen Meß- und Referenzstrahl bestimmt wird, wobei die überlagerte Strahlung detektiert und daraus mittels Fourier-Spektralanalyse unter Berücksichtigung der Weglängendifferenz der Referenzstrahlengänge der Abstand der Probenbereiche ermittelt wird.

Bei der Vermessung des Auges bietet es sich natürlich an, den Rückreflex von der Homhautvorderfläche auszuwerten, da dann auf besonders einfache Art und Weise die Augenlänge gemessen werden kann. Die Autokorrelationsfunktion des Referenzstrahls liefert bei der Auswertung einen ersten Bezugspunkt und die Überlagerung des Referenzstrahles mit dem Meßstrahl einen auf diesen Bezugspunkt referenzierten zweiten Meßpunkt, beispielsweise das Signal für den Augenhintergrund.

Bei der Vermessung des Auges ist es zweckmäßig, ein Fokussierelement in einem der Strahlengänge vorzusehen, um eine Fokussierung der beiden Strahlen sowohl auf der Cornea als auch am Fundus des Auges zu gewährleisten. Das Fokussierelement trägt also damit der fokussierenden Wirkung des Auges Rechnung, so daß der auf den Fundus fokussierte Meßstrahl als paralleles Strahlenbündel auf das Auge einfällt, wohingegen der andere Referenzstrahl bereits auf die Augenvorderfläche fokussiert ist. Natürlich kann das Fokussierelement im Meßstrahlengang (aufweitend) oder im Referenzstrahlengang (kollimierend) vorgesehen sein. Dieses Vorgehen erhöht die Signalstärke erheblich. Es ist deshalb zweckmäßig, daß der Referenz- oder der Meßstrahlengang ein vorzugsweise verstellbares Fokussierelement aufweist, um für Messungen am Auge den Meßstrahl auf die Augennetzhaut zu fokussieren. Für das analoge Verfahren ist es gleichermaßen vorteilhaft, daß für Messungen am Auge der Referenzstrahl mittels eines vorzugsweise verstellbaren Fokussierelement auf die Augenhornhaut fokussiert wird.

Beim Durchgang durch das Auge kann ein Dispersionseinfluß auftreten, der störend bemerkbar machen kann. Es ist deshalb zweckmäßig, eine entsprechende Dispersionskompensation vorzusehen. Zweckmäßigerweise ist deshalb in der Vorrichtung dafür gesorgt, daß der Meßstrahlengang oder der Referenzstrahlengang ein dispersionskompensierendes Element aufweist, um für Messungen am Auge Dispersionseinflüssen des Auges auf den Meßstrahl zu beeinflussen. Für das Verfahren gilt analog, daß für Messungen am Auge der Referenz- oder Meßstrahl mittels eines vorzugsweise verstellbaren dispersionskompensierendes Elementes hinsichtlich Dispersionseinflüssen des Auges beeinflußt wird.

Da die Fokussierung und die Dispersionskorrektur in einem engen Zusammenhang miteinander stehen, ist es günstig, die Verstellung des Fokussierelementes und die Einstellung des dispersionskorrigierenden Elementes miteinander zu koppeln, beispielsweise durch eine mechanische oder elektrische Kopplung, so daß eine synchrone Verstellung des dispersionskompensierenden Elementes und des Fokussierelementes bewirkt ist.

Die Erfindung erreicht also ein Interferometer, das mehrere Teilstrecken des Auges gleichzeitig mißt, indem eine getrennte Messung der separierten Meßbereiche erfolgt. Vorzugsweise ist diese getrennte Messung gleichzeitig durch entsprechende getrennte Überlagerung des Meßstrahles mit Strahlung aus verschiedenen Referenzstrahlengängen und getrenntem Nachweis der überlagerten Strahlung durchgerührt. Dabei können für die getrennten Nachweise separate Spektrometer verwendet werden, wenn, wie für einige Varianten der FD OCT, eine spektralselektive Detektion erfolgt.

Die erfindungsgemäße Vorrichtung bzw. das erfindungsgemäße Verfahren eignen sich, wie bereits erwähnt, besonders für die Vermessung des menschlichen Auges, aber auch andere teiltransparente Objekte können damit untersucht werden.

Erweitert man die Vorrichtung um eine Scanvorrichtung, die den Strahl quer über das Auge ablenkt, beispielsweise durch eine Parallelverschiebung oder eine Strahlablenkung, beispielsweise mittels Scan-Spiegeln, läßt sich ein dreidimensionales Probenbild erzeugen.

Mit dem erfindungsgemäßen Prinzip können mehrere Teilmessungen gleichzeitig quasi als "One-Shot"-Aufnahmen erfolgen oder auch Meßsequenzen mit vielen schnell aufeinander folgenden Einzelmessungen, die jeweils aus mehreren Teilmessungen bestehen, ausgeführt werden.

Zweckmäßigerweise wird die erfindungsgemäße Vorrichtung durch eine entsprechendes Steuergerät im Betrieb gesteuert. Dieses Steuergerät stellt dann den geschilderten Betrieb und insbesondere die Realisierung der geschilderten Betriebsweisen sicher. Vorteilhafterweise ist das Steuergerät auch geeignet ausgestaltet, beispielsweise durch einen Computer und entsprechende Programm-Mittel, um die Meßsignalaufbereitung aus den elektrischen Signalen des Detektors bzw. der Detektoren vorzunehmen, insbesondere die für FD OCT erforderliche Fourier-Transformation auszuführen.

Die Erfindung ermittelt von wenigstens zwei voneinander beabstandeten Probenbereichen bei einem transparenten und/oder diffusiven Gegenstand, z. B. bei einem Auge, in einer Meßzeit im Sub-Sekundenbereich die Abstände von Strukturen in den Probenbereichen. Hierzu wird vorzugsweise eine Anordnung gemäß einem Michelson-Interferometer verwendet. Im Interferometeraufbau wird kurzkohärente Strahlung verwendet, wobei beispielsweise die von einer Strahlquelle ausgehende kurzkohärente Strahlung in einen Meß- und einem Referenzstrahl aufgeteilt wird. Die verwendete Strahlung für Meßstrahl und Referenzstrahlen hat also im Vergleich zu den Probenbereichsabständen eine kurze Kohärenzlänge. Der Meßstrahl bestrahlt die Probenbereiche. Der Referenzstrahl wird in mindestens zwei räumlich getrennte Referenzstrahlengänge aufgeteilt, die den darin geführten Referenzstrahlen unterschiedliche Laufzeitänderungen aufprägen, wobei diese Laufzeitänderungen auf den Abstand der Probenbereiche vorabgestimmt sind. Die reflektierten Referenzstrählen werden dann getrennt interferierend mit den reflektierten und/oder rückgestreuten Meßstrahl vereinigt. Die vereinigten Strahlen werden detektiert und das detektierte Signal, wie bereits erwähnt, zur Distanzmessung fourierausgewertet.

Zur Messung des Abstandes der Meßbereiche bei einer transparenten und/oder diffusiven Probe, wie es zur Abstands-, Längen-, Dicken- und Profilmessung notwendig ist, wird die Probe mit einem Meßstrahl bestrahlt, und für jeden Probenbereich ist ein Referenzstrahl vorgesehen. Die Probenbereiche können sich dabei an verschiedenen Orten in Einfallsrichtung der optischen Strahlung befinden, wie auch seitlich versetzt zueinander. Der Laufzeitunterschied der Referenzstrahlengänge entspricht einem optischen Abstand der Probenbereiche in Bezug auf die Einfallsrichtung des Meßstrahls, wobei wenigstens einer der Probenbereiche wenigstens geringfügig (typischerweise mindestens $10^{-4}$ % der Strahlungsintensität) reflektiert und/oder rückstreut. Natürlich kann die Strahlkonfiguration des Meßstrahls auch über die Probe, insbesondere periodisch, bewegt werden, so daß die Probe quer zur Einfallsachse abgescannt wird. Damit können Profile der Probe vermessen werden. Anstatt nur einen Meßstrahl entlang einer optischen Achse einfallen zu lassen, kann man natürlich auch mehrere Meßstrahlen in einem Abstand nebeneinander einfallen lassen, um ein Oberflächenprofil schneller zu ermitteln.

Mit der erfindungsgemäßen Vorrichtung und dem erfindungsgemäßen Verfahren kann eine Probe hinsichtlich Abständen oder Profilen vermessen werden. Besonders bevorzugt ist der Einsatz bei einer optisch transparenten und/ oder diffusiven Probe, da dann auch die innere Probenstruktur gemessen werden kann. Die Weglängenunterschiede der räumlich getrennten Referenzstrahlengänge wird näherungsweise so eingestellt, daß sie dem zu erwartenden Abstand, einer zu bestimmenden Dicke, etc. bis auf eine gewisse Toleranz entspricht. Mit FD OCT wird dann nur noch durch Abweichung des nicht bekannten, noch zu bestimmenden Abstands vom voreingestellten Wert ermittelt. Soll z. B. die tatsächliche Länge eines menschlichen Auges gemessen werden, so weiß man bereits schön vorher, daß eine optische Länge von 34 mm plus/minus 4 mm zu erwarten ist. Man stellt also die Weglängendifferenz der Referenzstrahlengänge auf 34 mm ein, und die Signalauswertung der Fourier-Analyse ermittelt die Variation innerhalb des möglichen Bereiches von 8 mm. Natürlich ist es für die Vorrichtung bzw. das Verfahren ganz allgemein zweckmäßig, wenn die Weglängendifferenz zwischen den Referenzstrahlengängen während der Messung einstellbar ist bzw. eingestellt wird.

Mit der erfindungsgemäßen Vorrichtung bzw. dem erfindungsgemäßen Verfahren kann man am Auge neben der Augenlänge (zentral, peripher), auch die Vorderkammertiefe (zentral, peripher), die Comea-Dicke (zentral, peripher), die Tränenfilmdicke (zentral, peripher), die Linsendicke (zentral, peripher) und die Glaskörperdicke sowie entsprechende Oberflächenprofile (Topographien) der Cornea-Vorderfläche, der Comea-Rückfläche, der Linsenvorderfläche, der Linsenrückfläche und der Netzhaut gemessen werden. Ferner lassen sich durch geeignete Scanmechanismen Krümmungsradien, z: B. der Cornea-Vorderfläche, der-Cornea-Rückfläche, der Linsenvorderfläche und der Linsenrückfläche, bestimmen.

Natürlich kann ein Probenbereich auch mehrere interessante Teilbereiche umfassen. So kann man einen Probenbereich derart definieren, daß er die gesamte Augenvorderkammer umfaßt. Hierzu wird der Meßstrahl an einen Ort zwischen der Cornea-Vorderfläche und der Linsenrücktläche fokussiert. So kann dann die Reflexion an der Cornea-Vorderfläche, der Cornea-Rückftäche, der Linsenvorderfläche und der Linsenrückfläche innerhalb eines Probenbereiches detektiert werden. Die Distanz zwischen Cornea-Rückfläche und Linsenvorderfläche ist dann die Vorderkammertiefe. Bedingung dafür ist lediglich, daß der Meßbereich in dem Probenbereich so groß ist, daß ein Bereich von der Cornea-Vorderfläche bis zur Linsenrückfläche abgedeckt ist.

Die Erfinder erkannten weiter, daß bei einer bestimmten Ausgestaltung der Spektralanalyseeigenschaften die Fourier-Domain Kurzkohärenz-Interferometrie in der Lage ist, die gesamte Augenlänge des menschlichen Auges mit einer Messung zu erfassen, wenn bestimmte Parameter am Spektrometer eingehalten werden. Als wesentlicher Parameter stellte sich die Pixelzahl bzw. Anzahl empfindlicher Zellen des Detektor-Arrays heraus. Es ist deshalb in einer weiteren Erfindung eine Ausgestaltung der eingangs genannten Vorrichtung vorgesehen, bei der eine Spektrometeranordnung die überlagerten Strahlen detektiert, die ein die Strahlen spektral auffächerndes Element und ein Dektor-Array aufweist, das mindestens 7000 einzelne photoempfindliche Zellen aufweist. Der mit einem derartigen Detektor-Array erreichte Meßbereich ist beispielsweise bei einer Wellenlänge zwischen 700 und 900 nm sowie einer spektralen Bandbreite von 10 - 30 nm der verwendeten Strahlung so groß, daß damit die Augenlänge meßbar ist. Analog ist deshalb auch für das Verfahren der eingangs genannten Art vorgesehen, daß zur Detektion der überlagerten Strahlen eine Spektrometeranordnung verwendet wird, die ein die Strahlen spektral auffächerndes Element und ein Dektor-Array aufweist, das mindestens 7000 einzelne photoempfindliche Zellen aufweist.

Soweit in der voranstehenden oder nachfolgenden Beschreibung Verfahrensschritte, insbesondere Signalauswertungen, Ansteuerungen von verstellbaren Komponenten, wie beispielsweise einer durchstimmbaren Strahlungsquelle etc., erwähnt sind, können in den Vorrichtungen gemäß der Erfindung diese Verfahrensschritte durch die Steuereinrichtung vorgenommen werden, die dazu geeignete Betriebsmittel, beispielsweise eine Software-Steuerung umfaßt. Natürlich können die hier beschriebenen Merkmale auch in anderen Kombinationen als beschrieben realisiert werden. Insbesondere kann ein bestimmtes Merkmal auch ohne andere damit beschriebenen Merkmalen verwendet werden.

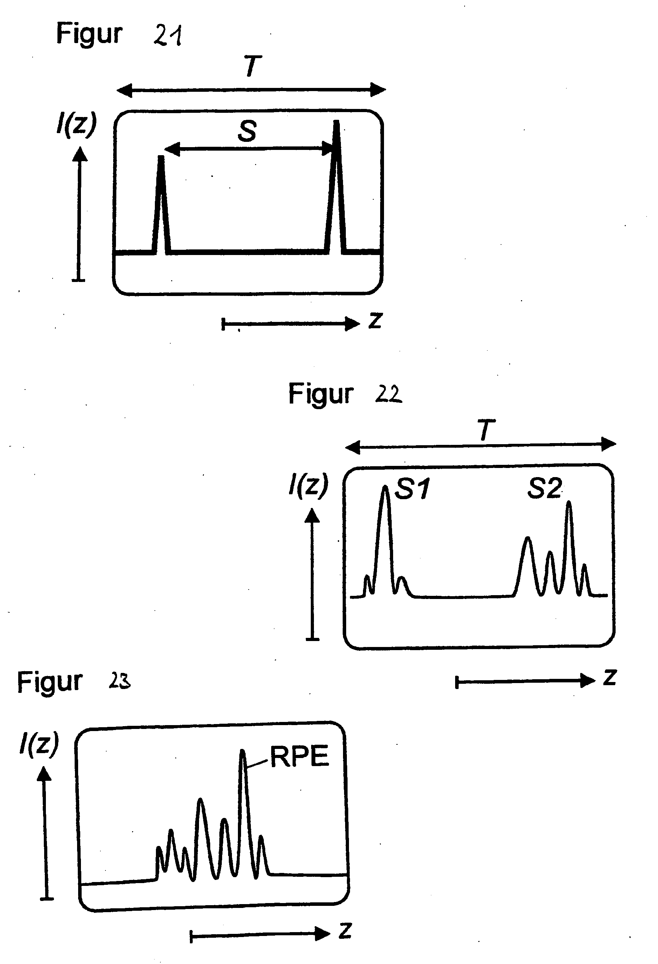

Dss Meßsignal der Kurzkohärenz-Interferometrie, das (in Analogie zum entsprechenden Ultraschall-Verfahren) sogenannte A-Scan-Signal, ist die Kreuzkorrelation des Referenzlichts mit dem Objektlicht am Interferometerausgang. Per Fouriertransformation einer spektralen Intensitätsverteilung bekommt man die Autokorrelation des zugrunde liegenden Lichtsignals. Bildet man die Fourier-Transformation (FT) des Lichtspektrums I(k) am Interferometerausgang, erhält man eine Autokorrelation der Summen der überlagerten Referenz- und Objektwellen. Diese Autokorrelation enthält auch die gewünschte Kreuzkorrelation oder das Interferogramm IN(z) des Referenzlichts mit dem Objektlicht. IN(z) ist dann das A-Scan Signal, das z.B. am Computer-Monitor dargestellt wird. Die Signalspitzen markieren die Positionen lichtreflektierender Stellen im Meßobjekt, wie Figur 21 zeigt. Wir können daher für das A-Scan-Signal vereinfacht schreiben:

$$IN(z) \sim FT\ \{I(k)\}$$

k ist die Wellenzahl, für die bekanntermaßen gilt: k = π/λ. z ist die Koordinate im Ortsbereich; IN(z) ist das Interferogramm und I(k) das Intensitätsspektrum des verwendeten Lichts. Im Stand der Technik wird in der Kurzkohärenz-Interferometrie meist Licht von Superlumineszenzdioden im nahen Infrarotbereich mit Wellenlängen-Bandbreiten um Δλ = 20nm benutzt. Dies ergibt eine Kohärenzlänge und damit eine Meßgenauigkeit in der Größenordnung von 30 μm. Die direkte Anwendung der FD OCT zur Augenlängen-Messung scheiterte daran, daß man mit den bisher erhältlichen Photodioden-Arrays nicht das gesamte der Augenlänge entsprechende Spektrum detektieren kann. Die Feldtiefe T (zuvor auch als Z bezeichnet), die ein Detektor-Array liefert, ist gegeben durch

$$T = N \cdot \pi/(2 \, \Delta k),$$

worin N die Pixel- oder Diodenanzahl des Photodioden-Arrays ist und Δk die Wellenzahl-Bandbreite des Lichts ist. Mit heute üblichen Anordnungen erreicht man Feldtiefen von etwa T ≈ 5 mm. Strecken größer T können offensichtlich nicht gemessen werden, wie man an Figur 21 leicht abliest.

Für die Messung der gesamten Augenlänge sind Meßfelder von 40 mm Tiefe und in Einzelfällen auch größer erforderlich. Dieses Problem umgeht beispielsweise die EP 1 602 320 A1 mit einem Interferometer, das - wie der erwähnte IOLMaster der Cart Zeiss Meditec AG - als Referenzfläche die Cornea benutzt und die Referenzstrahl-Meßstrahl-Wegdifferenz mittels eines flexiblen optischen Auszugs im Strahlengang verkleinert. So muß die Meßfenster-Tiefe nur die Abweichung zwischen der Referenzstrahl-Meßstrahl-Wegdifferenz und der Augenlänge zu erfassen. Dieses Prinzip ist jedoch auch nachteilig: Das für die Messung benutzte Spektrum am Interferometer-Ausgang basiert nun nämlich auf einem Interferogramm zweier Lichtwellen, die beide durch Reflexion an biologischen Grenzflächen (beispielsweise Cornea-Vorderfläche und Augenlinsen-Vorderfläche) entstehen. Man sieht ein Signal nur, wenn beide sehr instabilen Signale gleichzeitig vorhanden sind. Das ist nicht immer einfach zu realisieren; außerdem ist eine Optimierung der Intensitäten dieser zwei Wellen für maximale Sensitivität nicht einfach, da die für die Messung effektiven Reflektivitäten beider biologischer Grenzflächen auch von der Strahlposition abhängt, die wegen der inhärenten Bewegungen lebender Objekte schwer kontrollierbar ist. Schließlich ist die Sensitivität der FD OCT von der Position des Signals im. Meßfeld abhängig. Es können daher nicht beide eine zu messende Strecke markierenden Signale mit optimaler Sensitivität detektiert werden.

Die vorliegende Fourier-Domain-Interferometrie (FD OCT) zur Augen-Teilstreckenmessung verwendet vorzugsweise gleichzeitig zwei Meßfelder mit jeweils separatem zugehörigem Referenz- und Meßstrahl benutzt. Das Auge wird hierbei mit einem aus zwei gegeneinander axial versetzten Einzelstrahlen bestehenden Doppel-Meßstrahl beleuchtet und es werden zwei Referenzstrahlen benutzt.

Bei dieser "2-Meßfelder-Methode" werden über die zugehörigen Referenzspiegel die Meß-Positionen zweier Meßfelder in weiten Bereichen frei wählbar und es können gleichzeitig zwei separate Teilstrecken von der Ausdehnung des Meßfelds mit den darin enthaltenen Reflexen sichtbar gemacht werden. Sinnvollerweise wird man die Referenzstrahllängen so wählen, daß in den zwei Meßfeldern enthaltene Signal-Spitzen (S1 und S2) im Meßergebnis, also in der Fourier-Transformierten des Spektrums, nicht überlappen, sondern separat dargestellt werden, wie es in Figur 22 illustriert ist. Nun entspricht der Abstand der zwei Signal-Spitzen allerdings nicht mehr dem tatsächlichen Abstand der zugrunde liegenden reflektierenden Flächen, sondern ist um die Differenz der zwei Referenzstrahlen verkürzt. Man kann die Positionen der Meßflächen in Realzeit verfolgen und so erkennen, ob man eine sinnvolle Messung ausführt. Eine Identifizierung der Meßsignale ist ebenfalls aufgrund der Kopplung ihrer Positionen an die entsprechenden Referenzspiegel leicht erkennbar. Die nutzbare Feldtiefe T bzw. Z wird bei dem bekannten Ansatz auch dadurch begrenzt, daß das Kurzkohärenz-Interferogramm IN(z) aus der vom Detektor-Array registrierten reellen Intensität I(k) und nicht aus dem (komplexen) Frequenzspektrum j(k) des Lichts am Interferometer-Ausgang berechnet wird. Das Ergebnis ist eine Hermitesche Funktion: Man erhält nicht das Interferogramm IN(z), sondern die Autokorrelation der Summen der überlagerten Referenz- und Objektwellen und bei optimaler Position des Referenzspiegels zwei separate, zum Koordinaten-Ursprung symmetrische Rekonstruktionen des Meßsignals, nämlich eine bei positiven und eine bei negativen Koordinaten, was die Meßtiefe mindestens halbiert. Zu diesem Problem werden hier zwei Lösungen angegeben:

1. Man kann das komplexe Spektrum $\hat{\imath}(k)$ durch rechnerische komplexe Ergänzung der gemessenen reellen Intensität I(k) mit der zugehörigen Quadraturkomponente oder "Blindkomponente" rechnerisch a posteriori, also im Anschluß an die eigentliche Messung, gewinnen. Dadurch verschwindet die Rekonstruktion des Meßsignals bei negativen Koordinaten:

$$\hat{\imath}(k) = \tfrac{1}{2} \, [I(k) + i \cdot HT\{I(k)\}] \qquad (1)$$

HT {I} ist die Hilbert-Transformierte von / oder die zu / gehörige Quadraturkomponente.

2. Man kann die zur reellen Intensität $I(k)$ zugehörige Quadraturkomponente auch experimentell messen, indem man im Spektrum $I(k)$ eine Phasenverschiebung von 90° einführt. Die technische Lösung beruht nun auf geringen Verschiebungen des im Spektrometer benutzten Beugungsgitters.

Grundsätzlich kann man durch beide Lösungen die Feldtiefe des FD OCT verdoppeln und für geringere Feldtiefenansprüche auch mit einem Meßfeld auskommen. Jedoch muß man bedenken, daß der FD OCT tiefenabhängige Sensitivität besitzt. So ist die Sensitivität für jene Grenzfläche einer Strecke, die der virtuellen Referenzspiegel-Position am nächsten liegt, maximal. Sie kann aber für die hiervon maximal distanzierte Grenzfläche mehr als 10 dB geringer sein und eine Messung unterbinden. Ferner enthält das mit dem komplexen Spektrum berechnete A-Scan Signal immer noch störende Terme. Die Erfindung löst auch dieses Problem, weil jede Grenzfläche einer zu messenden Strecke durch eine entsprechende Referenzstrahl-Länge im zugehörigen Meßfeld mit maximaler Sensitivität dargestellt werden kann.

Bei den Messungen erhält man per Fourier-Transformation des Spektrums für jede lichtreflektierende Stelle im Auge eine Signalspitze. Die Zuordnung dieser Signale zu den tatsächlichen Augenstrukturen ist nicht immer einfach. Besonders die Signale der Retina können sehr komplex sein, wie es beispielsweise in Figur 23 dargestellt ist. Hierbei dominiert in der Regel das am retinalen Pigmentepithel (RPE) reflektierte Licht. Zur Augenlängenmessung wäre der Abstand dieses Signals vom vorderen Corneasignal geeignet. Allerdings kommt es auch vor, daß, je nach Position des Auges, andere Signalspitzen des retinalen Signalkomplexes dominieren, was zu Fehlmessungen führen kann. Um eindeutige Längenmessungen zu erhalten, gibt es die folgenden drei Möglichkeiten:

1. Man kann mehrmals messen, weil der FD OCT sehr schnell ist. Die Erfahrung zeigt, daß man dann meist auch Signale mit einer starken RPE-Spitze erhält. Man erkennt sie daran, daß sie bei den größten z-Positionen des Retina-Signalkomplexes auftritt.

2. Man kann die Signale mehrerer Messung summieren; dann erhält man eine Summe mit dominierender RPE Spitze.

3. Man kann die Tatsache benutzen, daß das vom RPE remittierte Licht in seiner Polarisation anders ist als Licht von den übrigen retinalen Schichten. Ein orthogonal zum Beleuchtungslicht polarisierter Referenzstrahl kann daher das von den übrigen retinalen Schichten reflektierte Licht stärker unterdrücken als jenes vom RPE.

Beleuchtet man das Auge, wie hier auch z. T. vorgesehen, mit einem Doppelstrahl, werden die Komponenten dieses Doppelstrahls an allen Grenzflächen des Auges zurück reflektiert. Dies führt zu reflektierten Wellen, deren Wegdifferenzen erheblich verkleinert werden, wenn deren Anfangs-Wegdifferenz nicht größer ist als zweimal Augenlänge plus Feldtiefe. Solche Wellen ergeben ein kontrastreiches Spektrum. Um die hierdurch erzeugten Artefakte zu vermeiden, sollte vorzugsweise die Anfangs-Wegdifferenz der Komponenten des beleuchtenden Doppelstrahls größer sein als zweimal Augenlänge plus Feldtiefe.

Die Erfindung wird nachfolgend unter Bezugnahme auf die Zeichnung beispielhalber noch näher erläutert. Es zeigen:

Fig.1               eine interferometrische Vorrichtung zur Vermessung eines Auges, wobei Meßstrahlung räumlich getrennt mit Strahlung aus drei Referenzstrahlengängen zur Interferenz gebracht wird,

Fig. 2              eine Darstellung ähnlich der Fig. 1 mit einer abgewandelten Spektrometer- Einheit,

Fig. 3              eine Darstellung ähnlich der Fig. 1, nun jedoch unter der Verwendung von Festoptiken anstelle Lichtleitfasern,

Fig. 4              eine Schemadarstellung zur Veranschaulichung der Wirkung eines Beugungsgitters des Interferometers der Fig. 1 - 3,

Fig. 5a - c         Ausführungsbeispiele für eine Optik mit geteilter Pupille, wie sie in den Interferometern der Fig. 1-3 zum Einsatz kommen könne,

Fig. 6              ein weiteres Interferometer ähnlich dem der Fig. 1, jedoch mit einer andersartigen räumlichen Trennung der Überlagerung des Meßstrahles mit den Referenzstrahlen,

Fig. 7              ein Interferometer ähnlich dem der Fig. 1, wobei die getrennte Überlagerung des Meßstrahls mit den Referenzstrahlen durch eine Polarisationstrennung erfolgt,

Fig. 8 und 9        Details einer möglichen Bauweise des Interferometers der Fig. 7,

Fig. 10             ein Interferometer ähnlich dem der Fig. 10, wobei die Trennung hier spektral erfolgt,

Fig. 11             eine Grafik zur Erläuterung der Wirkung der spektralen Trennung,

Fig. 12 und 13      Detailansichten von Komponenten, wie sie im Interferometer der Fig. 10 verwendet werden können,

Fig. 14             ein Interferometer ähnlich dem der Fig. 7, wobei die getrennte Überlagerung des Meßstrahls mit den Referenzstrahlen hiersequentiell erfolgt,

Fig. 15          ein Interferometer ähnlich dem der Fig. 7, wobei hier ein Doppelstrahl als Meßstrahl verwendet wird,

Fig. 16          eine Abwandlung des Interferometers der Fig. 15 mit nur einem Spektrometer,

Fig. 17          ein Spektrometer ähnlich dem der Fig. 15, jedoch ohne Polarisationstrennung aber mit Doppelstrahl,

Fig. 18          eine faseroptische Bauweise für das Spektrometer der Fig. 17,

Fig. 19          ein vereinfachtes Interferometer zur gleichzeitigen Detektion von in der Tiefe beabstandeten Bereichen,

Fig. 20          ein vereinfachtes Kurzkohärenz-Fourier-Domain-Interferometer zur Messung von zwei oder mehr in der Tiefe beabstandeten Bereichen,

Fig. 21          ein vereinfachter Meßsignalverlauf bei einer Tiefenmessung (A-Scan),

Fig. 22          ein vereinfachter Meßsignalverlauf bei einer Tiefenmessung mit einem komplexeren Signal,

Fig. 23          ein Meßsignalverlauf bei Messung der Augenretina,

Fig. 24          eine Vorrichtung zur Kurzkohärenz-Interferometrie mit einem Doppel-Meßstrahl und einem Doppel-Referenzstrahl,

Fig. 25          eine weitere Vorrichtung zur Kurzkohärenz-Interferometrie mit einem Doppel- Meßstraht und einem Doppel-Referenzstrahl,

Fig. 26          eine weitere Vorrichtung zur Kurzkohärenz-Interferometrie mit einem Doppel- Meßstrahl und einem Doppel-Referenzstrahl,

Fig. 27          eine weitere Vorrichtung zur Kurzkohärenz-Interferometrie mit einem Doppel- Meßstrahl und einem Doppel-Referenzstrahl und

Fig. 28          eine weitere Vorrichtung zur Kurzkohärenz-Interferometrie mit einem Doppel- Meßstrahl und einem Doppel-Referenzstrahl, in der exemplarisch verschiedene, ganz grundsätzlich mögliche Zusatzeinrichtungen vorhanden sind.

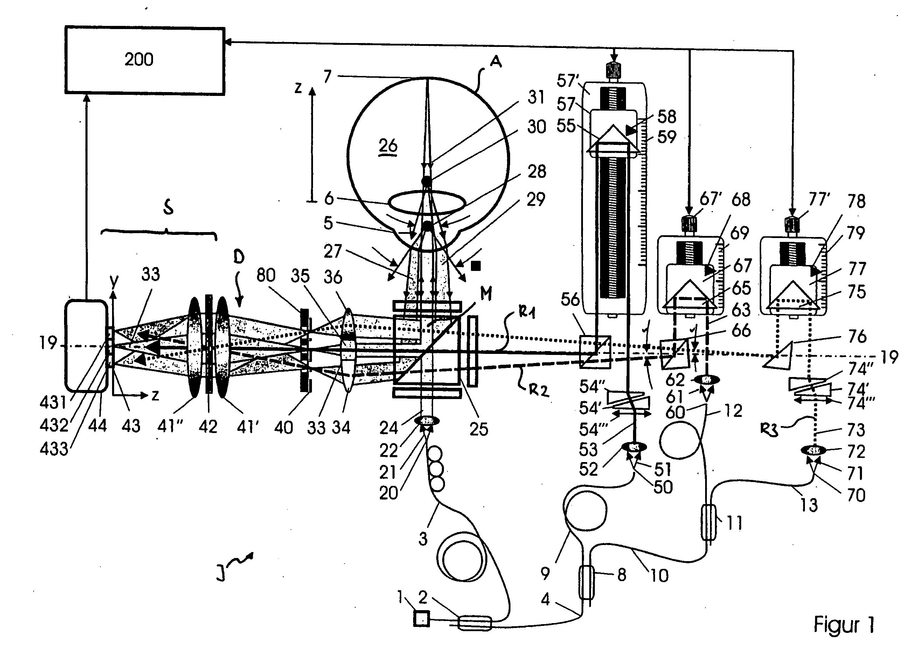

Figur 1 zeigt eine faseroptische Implementierungen eines Interferometers 1. Die remittierte Reflexstrahlung wird in einem Michelson-Aufbau mit mehreren pupillenmäßig getrennten Strahlengängen und jeweils zugehöriger Referenzstrahlen für verschiedene Augenstrukturen gleichzeitig registriert. Hierzu werden faseroptischer Koppler verwendet; es können auch andere faseroptische oder Freistrahl-Interferometerstrukturen benutzt werden, beispielsweise mit faseroptischen Zirkulatoren. Außerdem erfolgt die gleichzeitige Messung von drei Positionen der Augenstruktur (Corneavorderfläche, Linsenvorderfläche und Fundus). Man kann das Interferometer auch für die Messung von mehr oder weniger als drei Positionen abwandeln. In den Zeichnungen sind zur Vereinfachung oftmals nur die Strahlachsen eingezeichnet. Auch wird nachfolgend manchmal vereinfachend von "Strahl" gesprochen, anstatt von "Strahlenbündel" oder "Strahlenbüschel".

Von einer Kurzkohärenz-Lichtquelle 1, beispielsweise einer mit einer "Pig-Tail"-Faser ausgestatteten Superlumineszenzdiode oder von einer anderen Kurzkohärenz-Lichtquelle kommendes Licht wird von einem Faserkoppler 2 auf einen Interferometer-Meßarm 3 und einen Interferometer-Referenzarm 4 aufgeteilt. Um drei in unterschiedlichen Tiefenbereichen z des Auges liegenden Strukturen, nämlich Cornea 5, Augenlinse 6 und Augenfundus 7, mittels Fourier-Domain Kurzkohärenz-Interferometrie zu erfassen, werden simultan drei Referenzstrahlengänge R1, R2, R3 mit entsprechenden Strahlbündeln benutzt. Diese Strahlengänge werden aus der Referenzarm-Faser 4 über Faserkoppler abgeteilt: ein Koppler 8 teilt einen Fundus-Referenzstrahlengang. R1 in eine Faser 9. ab. Die verbleibende Strahlung in einer Faser 10 wird von einem Koppler 11 in eine Faser 12 für den Comea-Referenzstrahlengang R2 und eine Faser 13 für den Augenlinsen-Referenzstrahlengang R3 getrennt. Die Faserlängen für diese drei Referenzstrahlengänge sind so dimensioniert, daß an einem Photo-Detektorarray 43 trotz der kurzen Kohärenzlänge Interferenz mit dem jeweiligen aus den unterschiedlichen Objekttiefen kommenden Reflexstrahlbündel, nämlich von Cornea 5, Augenlinse 6 und Augenfundus 7, auftritt.

Die am Austrittspunkt 20 aus der Faser 3 austretende Beleuchtungsstrahlung 21 wird von einer Optik 22, beispielsweise einem Faser-Kollimator, kollimiert, durchläuft als paralleles Beleuchtungsstrahlbündel 24 einen Strahlteiler 25 und beleuchtet das Auge 26. Ein an der Vorderfläche der Cornea 5 reflektierter Comea-Reflexstrahl 27 kommt virtuell aus einem 1. Purkinje-Sanson-Bild 28 und ein von der Linsenvorderfläche reflektierte Augenlinsen-Reflexstrahlbündel 29 kommt virtuell aus einem 3. Purkinje-Sanson-Bild 30. Diese beiden Reflexstrahlbündel divergieren unter verschiedenen Winkeln. In der Figur 1 ist, um die Übersicht zu wahren, nur ein kleinerer Winkelbereich gezeichnet. Ein weiterer Reflex kommt vom Fundus 7 und bildet das reflektierte Fundus-Reflexstrahlbündel 31.

Die am Auge reflektierten Reflexstrahlbündel 27, 29 und 31 liegen somit als überlagerter Meßstrahl M vor und werden vom Strahlteiler 25 in einen Detektionszweig D und dort auf eine Relaisoptik 33 vor einem Spektrometer S gerichtet. Die Relaisoptik 33 paßt den Meßstrahl M, der ein Gemisch aus den drei Reflexstrahlbündeln ist, an das nachfolgende Spektrometer S an. Diese Optik besteht in dem Beispiel der Figur 1 aus drei Teiloptiken 34, 35 und 36 unterschiedlicher Brennweiten. Die Brennweiten sind so ausgelegt, daß die drei im Meßstrahl M enthaltenen, aus unterschiedlichen Tiefen am Auge reflektierten Reflexstrahlbündel in derselben Bildebene 40 vor dem Spektrometer S fokussiert sind; die Foki werden von den Spektrometeroptiken 41' und 41" über ein Beugungsgitter 42 auf ein Photo-Detektorarray 43 beispielsweise eine Array-Kamera 44 abgebildet. Die Spektrometeroptiken 41' und 41" können auch zu einer einzigen Optik zusammengefaßt vor oder hinter dem Beugungsgitter 42 aufgestellt werden.

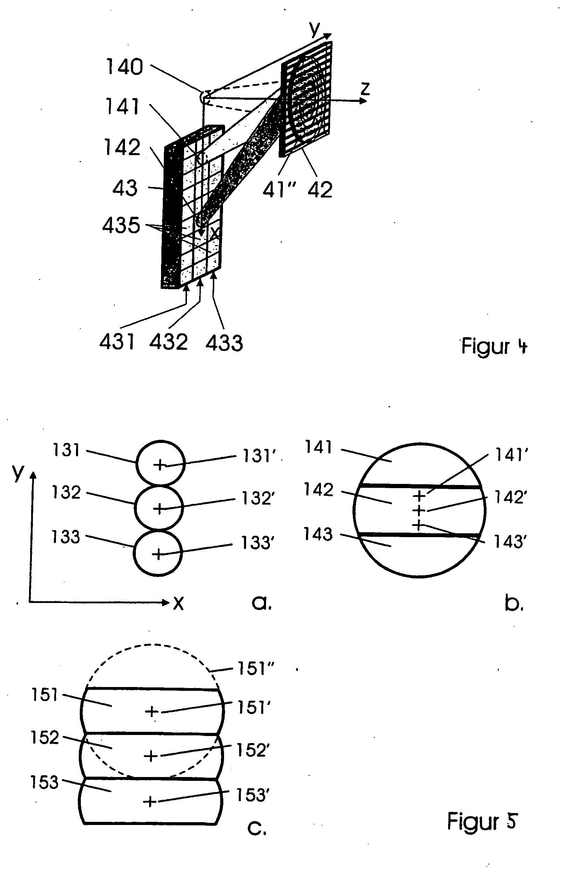

Wie in Figur 4 dargestellt ist, dispergiert das Beugungsgitter 42 die verschiedenen Wellenlängen des eintreffenden Lichts in x-Richtung auf Photodetektoren 435 des Photo-Detektorarray 43. In Figur 4 ist 140 ein von den Optiken 41' und 41" (siehe Figur 1) fokussiertes Lichtbündel nullter Beugungsordnung des Gitters 42; 141 und 142 sind von dem Beugungsgitter in x-Richtung dispergierte Lichtbündel erster Beugungsordnungen verschiedener Wellenlängen, die von den Optiken 41' und 41" auf die Photoempfänger des Arrays in eine Spalte 432 fokussiert werden. Den Lichtbündeln erster Beugungsordnung sind spektrale Komponenten des vom Fundus 7 remittierten Reflexstrahlbündels. 31 mit den entsprechenden spektralen Komponenten des zugehörigen Referenzstrahls 53 aus dem Referenzstrahlengang R1 überlagert. Die von Cornea 5 und Augenlinse 6 remittierten und ebenfalls mit Referenzlicht aus den ReferertzsVahlengängen R2 bzw. R3 überlagerten Reflexstrahlbündel 27 und 29 werden von den Optiken 41' und 41" auf benachbarte Array-Spalten 431 und 433 fokussiert.

Das am Austrittspunkt 50 aus der Faser 9 austretende Strahlbündel 51 wird von einer Optik 52 eines Faser-Kollimators kollimiert, durchläuft als paralleler Referenzstrahl 53 zwei Dispersions-Kompensationsprismen 54' und 54" und wird von einem Reflexionsprisma 55 über einen Strahlteiler 56 in Richtung einer optischen Achse 19 des Referenzarms 4 in das Interferometer I im ersten Referenzstrahleingang R1 eingespiegelt. Der Referenzstrahl 53 ist hier nur durch seinen Hauptstrahl gekennzeichnet. In der Figur 1 ist weiter angedeutet, daß dieser erste Referenzstrahl 53 auf dem Photo-Detektorarray 43 mit dem vom Fundus 7 kommenden Reflexstrahlbündel 31 überlagert ist. Durch Anpassung der optischen Länge des ersten ReferenzsVahlganges R1 vom Koppler 2 bis zum Strahlteiler 25 an die optische Länge vom Koppler 2 über den Fundus 7 des Auges A und zurück zum Strahlteiler 25 werden Interferenzen der überlappenden Strahlbündel sichergestellt; das bedeutet, daß der Ursprung des betreffenden Meßfelds am Fundus 7 liegt. Dies ist durch geeignete Wahl der Faserlängen und/oder der Position des Reflektionsprismas 55 eingestellt. Dazu ist vorzugsweise eine Verstellmechanik vorgesehen.

Diese kann beispielsweise wie in Fig. 1 ausgebildet sein. Das Reflektionsprisma 55 ist auf einem Tisch 57 einer manuell oder elektrisch betätigbaren Verschiebeeinheit 57' montiert. Eine Anpassung an unterschiedliche Augenlängen und Augenpositionen kann außerdem während einer Meßsequenz durch eine manuelle oder elektronisch angetriebene Verschiebung des Reflektionsprismas 55 mittels der Verschiebeeinheit 57' erfolgen. Die aktuelle Position des Prismas 55 kann mit Hilfe eines Zeigers 58 und eines Maßstabs 59 festgestellt werden. Alternativ können auch elektronische Positionsanzeigen benutzt werden und deren Daten können direkt in den Computer 200 eingegeben werden. Zur Kompensation der Probandenabhängigen Dispersion des Auges können die Prismen 54' und 54" relativ zueinander in Richtung des Doppelpfeils 54''' verschoben werden.

Das am Austrittspunkt 60 aus der Faser 12 austretende Strahlbündel 61 wird von einer Optik 62 eines Faser-Kollimators kollimiert und wird als paralleler Referenzstrahl 63 von einem Reflexionsprisma 65 über einen Strahlteiler 66 unter einem Winkel β zur optischen Achse 19 des Referenzarms 4 im zweiten Referenzstrahlengang R2 in den Strahlteiler 25 eingespiegelt. In Figur 1 ist angedeutet, daß die zweite Referenzstrahl 63 auf dem Photo-Detektorarray 43 mit dem von der Cornea 5 kommenden Retlexstrahlbündel 27 überlagert ist. Durch Anpassung der optischen Länge des zweiten Referenzstrahlengangs R2 vom Koppler 2 bis zum Strahlteiler 25 an die optische Länge vom Koppler 2 über die Cornea zum Strahlteiler 25 werden Kurzkohärenz-Interferenzen dieser zwei überlagernden Lichtbündel sichergestellt, beziehungsweise der Ursprung des Meßfelds festgelegt. Dies kann auch hier durch geeignete Wahl der Faserlängen und/oder der Position des Reflektionsprismas 65 erfolgen. Auch hier kann vorzugsweise eine Anpassung an unterschiedliche Augenlängen und Augenpositionen während einer Messung erfolgen, und zwar durch eine Verschiebung des Reflektionsprismas 65 mittels einer manuell oder elektrisch betätigten Verschiebeeinheit 67'. Die Position des Prismas 65 kann mit Hilfe eines Zeigers 68 und eines Maßstabs 69 festgestellt werden. Alternativ können auch hier elektronische Positionsanzeigen benutzt werden und deren Daten können direkt in den Computer 200 eingegeben werden.

Das am Austrittspunkt 70 aus der Faser 13 austretende Strahlbündel 71 wird von einer Optik 72 eines Faser-Kollimators kollimiert und als paralleler Referenzstrahl 73 nach Reflexion an einem Reflexionsprisma 75 von einem Reflexionsprisma 76 im dritten Referenzstrahlengang R3 unter dem Winkel a zur optischen Achse 19 des Referenzarms 4 in das Interferometer I eingespiegelt. In Figur 1 ist angedeutet, daß der dritte Referenzstrahl 73 auf dem Photo-Detektorarray 43 mit dem von der Vorderfläche der Augenlinse 6 beziehungsweise aus dem 3. Purkinje-Sanson Bild 30 kommenden Reflexstrahlbündel 29 überlagert. Durch Anpassung der optischen Länge des dritten Referenzstrahlenganges R3 vom Koppler 2 bis zum Strahlteiler 25 an die optische Länge vom Koppler 2 über die Vorderfläche der Augenlinse zum Strahlteiler 25 werden Kurzkohärenz-Interferenzen der überlagernden Lichtbündel sichergestellt, beziehungsweise die Position des Ursprungs dieses Meßfelds festgelegt. Dies erfolgt auch hier durch geeignete Wahl der Faserlängen und/oder der Position des Reflektionsprismas 75. Auch während einer Meßsequenz am Auge kann vorzugsweise eine Anpassung an unterschiedliche Augenlängen und Augenpositionen erfolgen, und zwar durch eine Verschiebung des Reflektionsprismas 75 mittels einer manuell oder elektrisch betätigten Verschiebeeinheit 77'. Auch hier kann die Position des Reflexionsprismas 75 mit Hilfe eines Zeigers 78 und eines Maßstabs 79 festgestellt werden. Alternativ können auch hier elektronische Positionsanzeigen benutzt werden und deren Daten können direkt in den Computer 200 eingegeben werden.

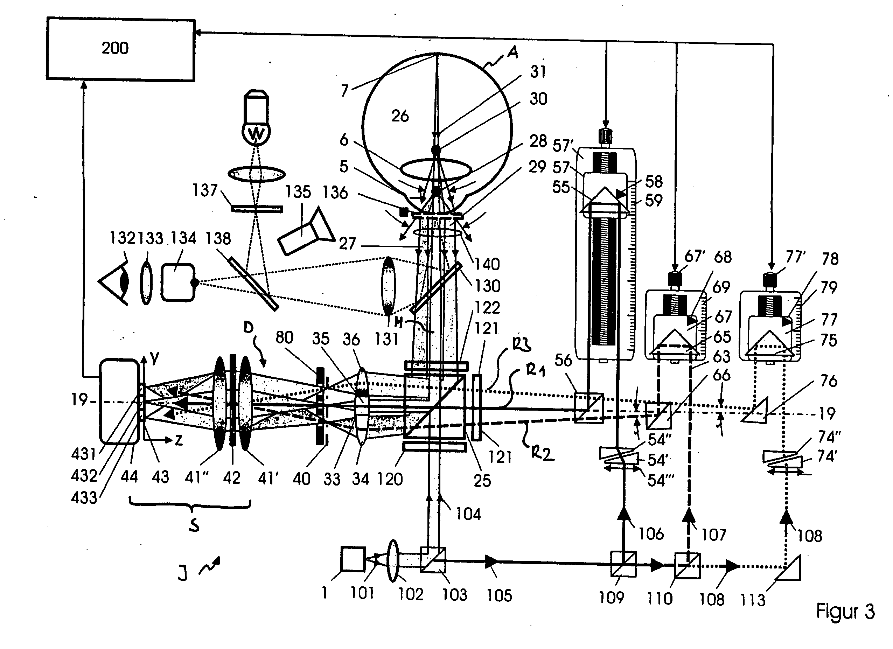

In Figur 3 ist Interferometer I dargestellt, das im wesentlichen dem Interferometer der Figur 1 entspricht, weshalb gleiche Elemente mit den gleichen Bezugszeichen versehen sind. Im Unterschied zur Figur 1 ist das Interferometer hier nun aber in Freistrahloptik ausgeführt. Ein von der Kurzkohärenz-Lichtquelle 1 emittierte Ursprungs-Strahlbündel 101, wird von einer Optik 102 kollimiert und trifft als Parallelstrahl auf einen Strahlteiler 103. Der Strahlteiler 103 teilt das Strahlbündel 101 auf ein Meßstrahlbündel 104 im Interferometer-Meßarm 3 und ein Strahlbündel 105 im Interferometer-Referenzarm 4 auf. Das Strahlbündel 105 ist hier wieder durch seinen Hauptstrahl angedeutet.

Das vom Strahlteiler 103 reflektierte Beleuchtungsstrahlbündel 104 durchläuft den Strahlteiler 25 und beleuchtet das Auge 26. Zurück läuft ein Meßstrahl M, der wiederum ein Gemisch aus folgenden Reflexstrahlbündeln enthält: das an der Corneavorderfläche reflektierte Reflexstrahlbündel 27 kommt virtuell aus dem 1. Purkinje-Sanson-Bild 28 und das von der Linsenvorderfläche reflektierte Augenlinsen-Reflexstrahlbündel 29 kommt virtuell aus dem 3. Purkinje-Sanson-Bild 30. Diese beiden Reflexstrahlbündel werden divergent reflektiert und sind entsprechend aufgeweitet. In der Figur 3 ist von diesen beiden Reflexstrahlbündeln wieder nur ein kleiner Winkelbereich gezeichnet. Ein weiterer Reflex kommt vom Fundus 7 und bildet das Fundus-Reflexstrahlbündel 31. Insoweit liegen identische Verhältnisse wie bei dem Interferometer I der Figur 1 vor. Dies gilt auch für die Detektion der überlagerten Strahlen im Detektionzweig D.

Für die drei in unterschiedlichen Tiefenbereichen des Auges befindlichen Strukturen, beispielsweise Cornea 5, Augenlinse 6 und Fundus 7, werden, wie im faseroptischen Interferometer I der Figur 1, drei Referenzstrahlgänge R1, R2, R3 benutzt. Diese werden hier mit Hilfe von Strahlteiler 109 und 110 erzeugt. Ansonsten ist die Einkopplung und Weglängenverstellung unverändert.

Im Interferometer **I** der Figur 3 ist zur Beobachtung der Position des Probandenauges 26 relativ zum Fundus-Reflexstrahlenbündel 31 noch eine Beobachtungsvorrichtung bestehend aus einem teildurchlässigen Spiegel 130 und einer Optik 131 angeordnet. Die Beobachtung des Probandenauges kann dann direkt (132), mit Hilfe eines Okulars 133 oder mit Hilfe einer Kamera 134 erfolgen. Es kann auch sinnvoll sein, das Probandenauge 26 zusätzlich mit einer inkohärenten Lichtquelle 135 zu befeuchten. Ferner kann zur präzisen Positionierung des Probandenauges ein Bild 136 einer Strichplatte 137 benutzt werden, welches über teildurchlässige Spiegel 138 und 130 auf die Cornea 5 projiziert wird.

Bei Fehlsichtigkeit können dem Auge 26 sammelnde oder zerstreuende Hilfsoptiken 140 vorgesetzt werden, welche die Fehlsichtigkeit kompensieren.

Es sei noch erwähnt, daß man zur Vermeidung von störenden Reflexionen und zur Optimierung der Strahlintensitäten das auf den Strahlteiler 25 treffende Beleuchtungsstrahlbündel 104 mittels eines Polarisators 120 linear polarisieren kann und den Strahlteiler 25 als polarisierenden Strahlteiler ausbilden kann. Unter Verwendung weiterer polarisationsoptischer Komponenten wie $\lambda/4$-Platten in den Positionen 121 und 122 können dem Stand der Technik entsprechend Reflexionsverluste von Referenz- und Reflexstrahlbündel beim mehrmaligen Durchgang durch den Strahlteiler weitgehend- vermieden werden. Solche in der technischen Optik bekannte Methoden können auch für die Strahlteiler 56 und 66 benutzt werden.

Die oben beschriebene Beobachtungsvorrichtung, bestehend aus dem teildurchlässigem Spiegel 130 und der Optik 131 sowie der Hilfsoptik 140 zur Kompensation von Ametropien des Probandenauges, können auch im Interferometer I der Figur 1 eingesetzt werden. Ebenso kann dort auch zur Vermeidung von störenden Reflexionen und zur Optimierung der Strahlintensitäten das auf den Strahlteiler 25 treffende Meßstrahlbündel 24 mittels des Polarisators 120 linear polarisiert werden, der Strahlteiler 25 als polarisierender Strahlteiler ausgebildet werden, und $\lambda/4$-Platten in den Positionen 121 und 122 angeordnet werden.

Die Relaisoptik 33 kann im einfachsten Fall aus drei kreisrunden Teiloptiken 131, 132 und 133 unterschiedlicher Brechkräfte aufgebaut sein, wie das in der Figur 5a angedeutet ist. 131', 132' und 133' sind die Durchstoßpositionen der zugehörigen optischen Achsen. Die Teiloptiken sind so anzuordnen, daß ihre optischen Achsen in der y-z-Ebene liegen, entsprechend der Auffächerung der Referenzstrahlen in der y-z-Ebene. Alternativ können diese drei Teiloptiken zur Erhöhung ihrer Lichtleitwerte auch aus Teilen größerer kreisrunder Optiken unterschiedlicher Brechkräfte zusammengesetzt werden, wie in Figur 5b skizziert ist. Den Optiken 131, 132 und 133 der Figur 5a entsprechen hier die Teiloptiken 141, 142, und 143; die Durchstoßpunkte der optischen Achsen 141' und 143' können hier auch außerhalb der zugehörigen Teiloptiken liegen. Schließlich können die drei Teiloptiken auch aus zentralen Ausschnitten größerer kreisrunder Optiken unterschiedlicher Brechkräfte zusammengesetzt werden, wie es in Figur 5c abgebildet ist. Hier sind Optiken 151, 152 und 153 als zentrale Ausschnitte größerer Optiken ausgeführt (wie für 151 mit dem Kreis 151" angedeutet). 151', 152' und 153' sind die Positionen die Durchstoßpunkte der zugehörigen optischen Achsen.

Grundsätzlich können die Teiloptiken der Relaisoptik 33 auch an verschiedenen z-Positionen der optischen Interferometerachse 19 positioniert sein. Es muß dann nur durch entsprechende Wahl ihrer Brennweiten gewährleistet sein, daß die drei Reflexstrahlbündel 27, 29 und 31 in einer gemeinsamen Ebene 40 vor dem Spektrometer fokussiert werden.

Das Fourier-Domain Kurzkohärenz-Interferometer I muß wegen der separaten Reflexstrahlen kalibriert werden.

Die Fourier-Domain Kurzkohärenz-Interferometrie liefert als Meßergebnis die optische Distanz der Objekt-Meßstelle relativ zur "Wegdifferenz-Null-Position" (für diese ist die optische Länge des Meßstrahls gleich der des Referenzstrahls). Die Distanzen der Ursprünge der voneinander unabhängigen Reflexstrahlengänge mit Referenzstrahlen R1, R2, R3 müssen daher festgelegt werden. Außerdem ist der Meßbereich in der Tiefe begrenzt, bei den eingangs angenommenen Parametern beispielsweise auf rund 5,3 mm; das Interferometer I muß daher auch an die zu erwartenden Augendistanzen grob vorangepaßt werden. Zur Anpassung und Kalibrierung kann beispielsweise als Grundeinstellung ein Planspiegel als Objekt im Meßstrahlengang an der zu erwartenden Position der Cornea positioniert werden. Als nächstes werden dann alle Reflexionsprismen (55, 65 und 75) so positioniert, daß alle zugehörigen Referenzstrahlen 53, 63, 73 Kurzkohärenz-Interferenzen mit dem von dieser Planplatte reflektierten Lichtbündel zeigen. Von dieser Grundjustierung ausgehend, können beispielsweise die Positionen je eines Referenzspiegel in die zu erwartenden Positionen der zu messenden Linsenvorderfläche 6 und des Fundus 7 eingestellt werden. Durch Ablesen der ausgeführten Verschiebungen mittels der Meßvorrichtungen 58, 59 sowie 68, 69 und 78, 79 oder der entsprechenden elektronischen Positionssignale hat man einen Basiswert für zu messenden Längen. Die optische Messung gibt nun die Distanz der tatsächlichen Position der Reflexionsstellen im Auge relativ zur Basisposition. Addiert man diese zum Basiswert, hat man die gesuchte Distanz in hoher Genauigkeit.

Die drei vom Auge remittierten Reflexstrahlbündel 27, 29 und 31 beleuchten neben den zugehörigen Teiloptiken 34, 35 und 36 auch die jeweils anderen Teiloptiken und werden von diesen defokussiert auf das Detektorarray 43 treffen. Es kommt dadurch zu Falschlicht und damit zu einem unerwünschten Untergrund. Da dieses Falschlicht mit den dort fokussierten Referenzstrahlen 53, 63, 73 bezüglich Wegdifferenz nicht angepaßt ist, entstehen am Detektorarray sehr hohe Modulationsfrequenzen, die vom Detektorarray nicht aufgelöst werden. Dennoch kann es neben dem erhöhten Rauschen durch Aliasing zu zusätzlichen Fehlsignalen kommen. Es ist daher vorteilhaft, dieses Falschlicht so weit wie möglich zu unterdrücken. Das ist durch eine räumliche Filterung in der Bildebene 40 möglich. Hierzu wird in dieser Ebene eine Lochblendenmaske 80 angebracht, mit drei Öffnungen an den Stellen der Bündelfoki. Die Positionen der Bündelfoki hängen allerdings - mit Ausnahme des Fundusbündel-Fokus - von der Position des Auges 26 ab. Das Auge muß daher mittels der oben beschriebenen Vorrichtung zur Beobachtung der Position des Probandenauges positioniert werden. So können die Reflexstrahlbündel von Fundus 7 und Cornea 5 gut diskriminiert werden.

Die Figuren 1 und 3 zeigen die Messung der Positionen von Comeavorderfläche, Linsenvorderfläche und Fundus. Wie schon eingangs erwähnt, können jedoch noch weitere Positionen von Augenstrukturen gleichzeitig gemessen werden, beispielsweise die Linsenrückfläche mit Hilfe des vom 4. Purkinje-Sanson Bild virtuell remittierten Lichts oder die Position der Cornea-Rückfläche mit Hilfe des vom 2. Purkinje-Sanson virtuell remittierten Lichts. Es bedarf hierzu entsprechender zusätzlicher Referenzstrahlbündel und Relais-Teiloptiken (33) sowie zusätzlicher Öffnungen in der Lochblendenmaske 80 und weiterer Arrayspalten. Allerdings kann man bei Meßfeldtiefen um die 5 mm ost davon ausgehen, daß die beiden Cornea-Positionen im Meßsignal gleichzeitig vorhanden sind.

Schließlich kann man die beschriebenen Interferometer auch zur Messung anderer Positionen wie etwa der Linsenrückfläche modifizieren. Hierzu muß z. B. der Referenzstrahl 53 entsprechend verkürzt werden und die Brennweite der Optik 35 verkleinert werden.

Es sei noch erwähnt, daß die Verwendung eines Arrays 43 mit nur drei (431, 432 und 433) oder vier Spalten nicht einschränkend zu verstehen ist. Kommerziell erhältliche Array-Kameras besitzen oft mehrere hundert Spalten. Diese können auf zweierlei Weise benutzt werden: man kann einerseits zwischen den auszulesenden Spalten mehrere ungenutzt lassen und damit optisches und elektronisches Übersprechen unterbinden. Es können aber auch die Zeilenelemente mehrerer benachbarter Spalten durch Binning miteinander verbunden werden, um die Meßempfindlichkeit zu erhöhen.

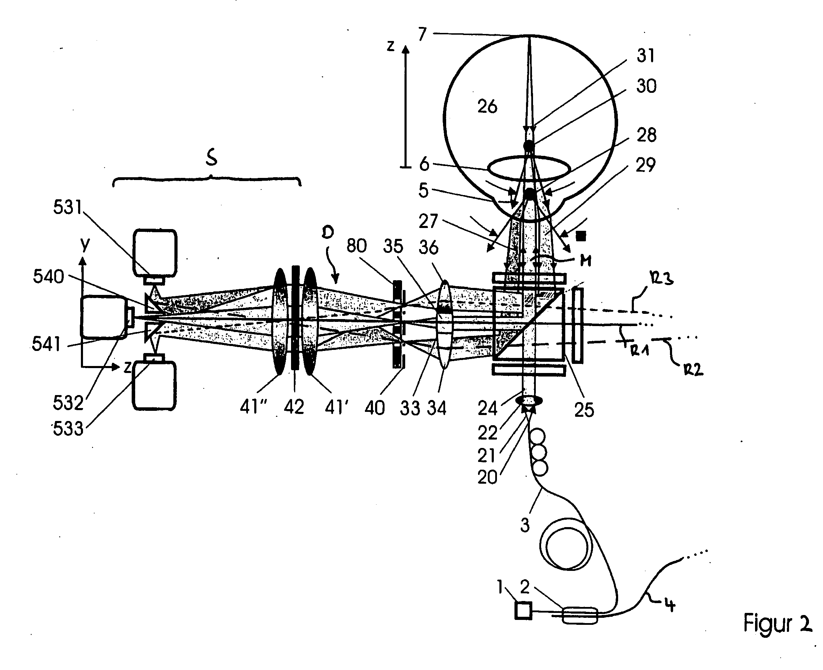

Figur 2 zeigt eine Abwandlung des Interferometer I der Figur 1; unverändert oder funktionsgleich aus Figur 1 übernommene Elemente sind deshalb mit denselben Bezugszeichen gekennzeichnet und werden hier nicht noch einmal erläutert. Der Unterschied zwischen der Bauweise der Figuren 1 und 2 liegt im wesentlichen darin, daß das Spektrometer S nun nicht mehr ein zweidimensionales Photo-Dekektorarray verwendet, sondern drei einzelne Zeilen-Photo-Detektorarrays 531, 532 und 533. Zur Aufteilung der z. B. durch Pupillentrennung räumlich getrennten mit den Referenzstrahlen 53, 63, 73 überlagerten Reflexstrahlenbündeln 31, 29, 27 sind im Detektionszweig D zwei geeignete Spiegel 540, 541 vorgesehen, die zwei der mit Referenzstrahlen überlagerten Reflexstrahlbündel zu zwei senkrecht zur optischen Achse liegenden Photo-Detektorarrayzeilen 531 und 533 umlenken.

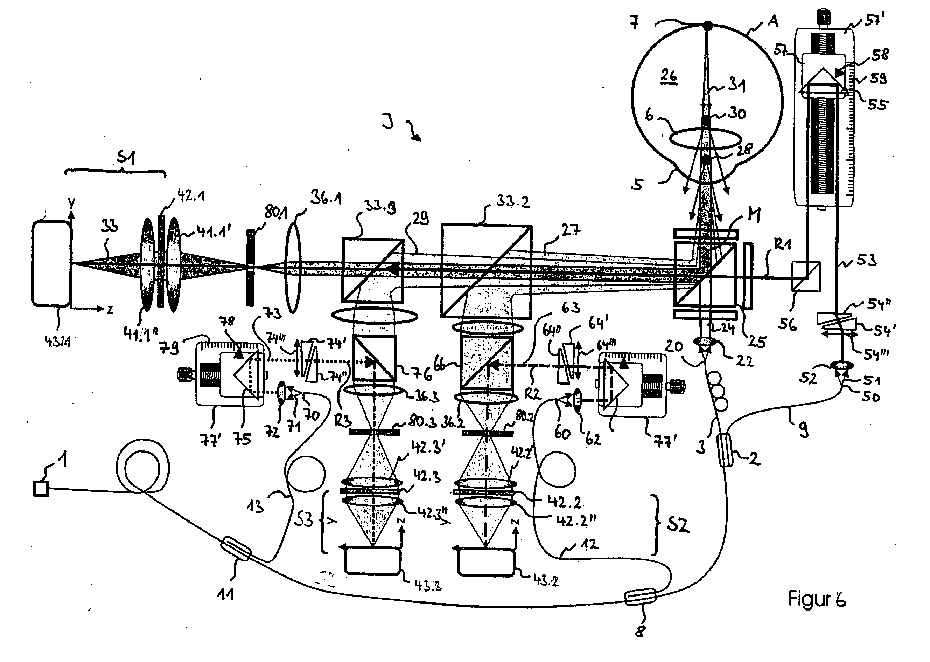

Ein nochmals abgewandeltes Interferometer I ist in Figur 6 gezeigt. Auch hier sind unverändert oder funktionsgleich aus Figur 1 übernommene Bauteile mit denselben Bezugszeichen versehen, so daß die Wiederholung der Beschreibung entbehrlich ist. Das Interferometer I der Figur 6 verwendet eine gegenüber der Figur 1 abweichende Art der räumlichen Auftrennung. Die Auftrennung des Meßstrahls M und die Überlagerung mit den drei Referenzstrahlen 53, 63, 73 erfolgt hier durch Strahlteiler. Bauteile, die in ihrer Funktion oder ihrem Aufbau dem der Figur 1 entsprechen, sind mit denselben Bezugszeichen versehen, wobei gegebenenfalls zur Unterscheidung der Bauteile für die drei einzelnen Überlagerung ein Suffix .1, .2 oder .3 angehängt wurde. Das Bauteil 42.3 entspricht also beispielsweise dem Bauteil 42 der Figur 1, esist jedoch in der Darstellung der Figur 6 lediglich für die Überlagerung mit dem Referenzstrahl R3 wirksam.

Die getrennte Überlagerung der Reflexstrahlbündel 27, 29, 31 im Meßstrahl M erfolgt in der Bauweise der Figur 6 nun nicht durch eine mit geteilter Pupille versehenen Relaisoptik 33, sondern durch Umlenkelemente 33.1- sowie 33.2, die.die Reflexstrahlbündel 27 und 29 aus dem Strahl auskoppeln. Die Umlenkelemente 33.2 und 33 können dabei beispielshalber als spektral neutrale Strahlteiler ausgeführt werden.

Das Umlenkelement 33.2 leitet einen Teil des Meßstrahles M aus dem Strahlbündel, das vom Strahlteiler 25 abgeteilt wurde, aus und stellt es zur Überlagerung mit dem zweiten Referenzstrahl 63 bereit, der separat über einen Strahlteiler 66 eingekoppelt wird. Die nachgeschaltete Optik entspricht im wesentlichen der Optik des Detektorzweigs D der Figur 1, mit dem Unterschied, daß nun keine räumliche getrennte Pupille vorliegt; der Strahlengang nach dem Strahlteiler 33.2 führt keine räumliche getrennten Strahlen mehr. Dementsprechend muß das Photo-Detektorarray 43.2 auch nicht zweidimensional ausgeführt sein.

Gleiches gilt für das Photo-Detektorarray 43.3, das am Ende des Strahlengangs nach der Abteilung durch den Strahlteiler 33.3 liegt.

Der nach den Strahlteilern 33.2 und 33.3 noch vorhandene Anteil des Meßstrahles M wird mit dem Referenzstrahl 53 überlagert und am Photo-Detektorarray 43.1 nach zu voriger spektraler Auftrennung nachgewiesen.

Das Interferometer der Figur 6 weist also drei Spektrometer auf, S1 für die Überlagerung des verbleibenden Meßstrahls M mit dem Referenzstrahl 53 aus Referenzstrahlengang R1, das Spektrometer S2 im durch den Strahlteiler 33.2 abgetrennten Teil des Strahlengangs, in den der Referenzstrahl 63 eingekoppelt wird, und das Spektrometer S3, das dem Strahlteiler 33.3 nachgeschaltet ist und die abgeteilte Strahlung überlagert mit dem Referenzstrahl 73 nachweist.

Die räumliche Abtrennung durch Strahlteiler hat den Vorteil, daß die Lochblenden 80.1, 80.2 und 80.3 sehr viel wirksamer auf die Falschlichtunterdrückung ausgelegt werden können. Auch ist eine separate Fokussierung mittels der nun einfacher eigenständig verstellbaren Teiloptiken 36.1, 36.2 und 36.3 ohne Aufwand möglich, wodurch das Signal/Rausch-Verhältnis für die in der Tiefe separierten Objektbereiche verbessert ist.

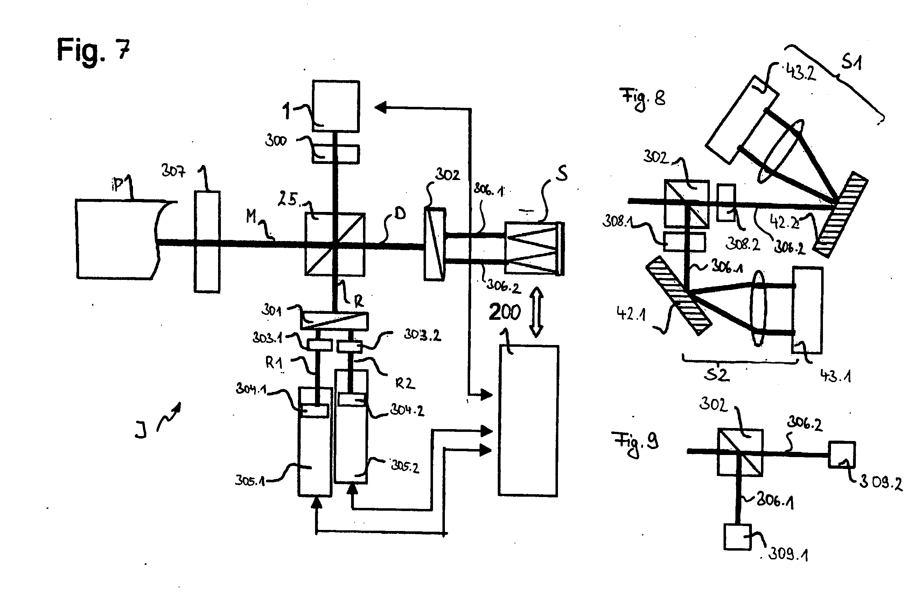

Figur 7 zeigt ein Interferometer **I,** das wie die zuvor beschriebenen Interferometer auch das Prinzip verfolgt, in unterschiedlich langen Referenzstrahlengängen R1, R2 geführte Referenzstrahlen mit einem Meßstrahl M eigenständig zu überlagern und nachzuweisen. Die anhand der zuvor geschilderten Figuren realisierten Details sind deshalb auch uneingeschränkt für die nachfolgend beschriebenen Varianten möglich. Auch sind übereinstimmende Elemente mit denselben Bezugszeichen versehen, so daß auf Ihre Erläuterung hier verzichtet werden kann. Ebenso ist in den nachfolgenden Figuren durch Anhängung eines entsprechendes Suffixes .1, .2 etc. eine Unterscheidung hinsichtlich der Referenzstrahlen und deren Strahlengänge bzw. -überlagerungen getroffen.

Das Interferometer I der Figur 7 nimmt eine Trennung der Referenzstrahlengänge hinsichtlich der Polarisation vor. Dazu wird die Strahlung der Lichtquelle 1 mittels eines Polarisators 300 zirkular polarisiert, bevor sie auf den Strahlteiler 25 und von dort eine Probe P fällt. Alternativ wird eine zirkular polarisiertes Licht abgebende Strahlquelle verwendet. Im Referenzstrahlengang R wird die zirkular polarisierte Strahlung mittels eines Polteilers 301 in zwei senkrecht zueinander polarisierte Strahlen aufgeteilt. Der Polteiler kann beispielsweise als Wollaston-Prisma ausgeführt werden. Einstellbare Graufilter 303.1 bzw. 303.2 erlauben eine Einstellung der Intensität der Strahlung in den zwei Referenzstrahlgängen R1, R2, was zur Optimierung des Meßsignals vorteilhaft ist. Die Referenzstrahlen in R1 und R2 werden an Reflektoren 304.1 bzw. 304.2 reflektiert. Jeder Reflektor 304 ist auf einem Schlitten 305 verschieblich befestigt, so daß die Weglänge des Referenzstrahlengangs individuell einstellbar ist. Dies kann unter Steuerung des Computers 200 erfolgen. Die Verstellmechanik kann dabei insbesondere den anhand der Figuren 1ff geschilderten Aufbau haben. Der überlagerte Meßstrahl M mit den Referenzstrahlen, die in Rückreflexionsrichtung des Referenzstrahlengangs R1 und R2 nach dem Polteiler 301 überlagert sind, erfolgt im Detektionszweig D.

Darin werden die überlagerten Strahlen wieder durch einen Polteiler 302 aufgeteilt, so daß überlagerte Strahlen 306.1 und 306.2 vorliegt, in denen der Meßstrahl M mit dem Referenzstrahl aus R1 bzw. R2 überlagert ist. Die unterschiedliche Weglänge der Referenzstrahlengängen R1 und R2 bewirkt dabei, wie zuvor bereits beschrieben, eine entsprechende Tiefenselektion in der Probe P, die beispielsweise wiederum das menschliche Auge A sein kann. Die derart nach ihrer Polarisation getrennten überlagerten Strahlen 306 werden dann in einem Spektrometer S getrennt nachgewiesen.

Zur räumlichen Vermessung der Probe P ist schließlich noch ein Scanner 307 mit geeigneter Optik vorgesehen, der die Probe mit der einfallenden Strahlung abscant.

Der mögliche Aufbau des Spektrometers S ist in Figur 8 exemplarisch dargestellt. Dem Polteiler 302 ist beispielsweise jeweils ein Polarisationsmanipulator 308.1 bzw. 308.2 nachgeordnet, mit dem man die Polarisationsrichtung der Strahlen so drehen bzw. einstellen kann, daß sich im nachgeordneten Strahlengang eine maximale Ausbeute einstellt. Die polarisationsgetrennten Strahlen 306.1 und 306.2 fallen nach Durchlauf durch den Polarisationsmanipulator 308.1 bzw. 308.2 auf das Beugungsgitter 42.1 bzw. 42.2, Reflexionsbeugungsgitter. Die so erhaltene spektrale Aufteilung wird dann in Detektorarrayzeilen 43.1 bzw. 43.2.nachgewiesen.

Das Spektrometer S ist aus zwei Teil-Spektrometern S1 und S2 aufgebaut, die die polarisationsgeteilte überlagerte Strahlung 306 individuell nachweisen.

Die in den Figuren 1-6 durch räumliche Trennung realisierte getrennte Überlagerung und Detektion ist in der Bauweise der Figur 7 durch eine Polarisationstrennung erreicht.

FD OCT kann, wie erwähnt, auf zwei Weisen arbeiten. Zum einen kann eine kurz-kohärente Strahlquelle verwendet werden, die es vorstehend beschrieben wurde. Dann ist eine spektrale Auftrennung der überlagerten Strahlung erforderlich. Zum anderen kann auch eine durchstimmbare kurz-kohärente Strahlungsquelle verwendet werden. Stimmt man den Spektralbereich dieser Quelle durch, muß keine spektrale Analyse der Strahlung mehr erfolgen; statt dessen kann ein spektral unempfindlicher Detektor verwendet werden. Die Probenstruktur kann in beiden Fällen durch Bildung der inversen Fouriertransformierten des spektralen Interferenzmusters erhalten werden. Natürlich können die zuvor und auch nachfolgend erläuterten Interferometer I für eine der beiden Arbeitsweisen angepaßt werden. Die nötigen Abwandlungen sind in Figur 9 exemplarisch für das Interferometer I der Figur 7 gezeigt. Dem Polarisationsstrahlteiler 302 sind anstelle der Spektrometer S1 und S2 nun lediglich individuelle Detektoren 309.1 und 309.2 nachzuordnen, die keine spektrale Analyse der einfallenden Strahlung vornehmen. Die Bauweise der Figur 7 kann in der Variante gemäß Figur 9 auch für-TD OCT eingesetzt werden. Hier ist lediglich eine synchronisierte Verstellung der Weglänge der Referenzstrahlengänge R1, R2 erforderlich und das Durchstimmen der Lichtquelle 1 entfällt.

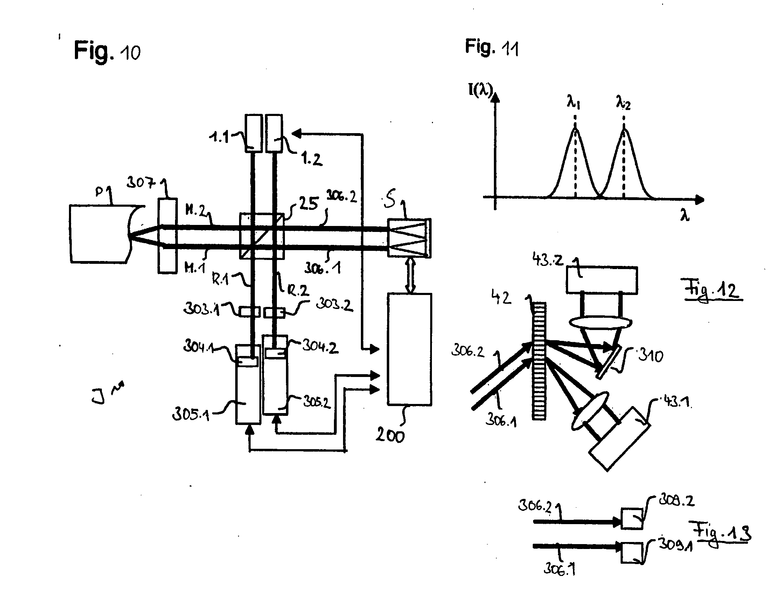

Figur 10 zeigt eine Abwandlung der Bauweise der Figur 7, bei der die räumliche Trennung der Referenzstrahlung R1 und R2 und die entsprechende Überlagerung nun nicht gemäß der Polarisation erfolgt, sondern durch spektrale Trennung. Es sind deshalb zwei Lichtquellen 1.1 und 1.2 vorgesehen, die Strahlung bei einer unterschiedlichen Wellenlänge abgeben, wie dies in Figur 11 gezeigt ist. Im dort aufgetragenen Spektrum ist die linke Wellenlängenverteilung beispielsweise der Lichtquelle 1.1 zuzuordnen, die rechte Wellenlängenverteilung ist in der Strahlung der Lichtquelle 1.2 gegeben. Die Bauweise des Interferometers I der Figur 10 entspricht ansonsten der dem Interferometer der Figur 7, mit dem Unterschied, daß nun die Polteiler nicht mehr erforderlich sind, sondern zwei spektral getrennte Strahlen durch das Interferometer propagieren. Die der Übersichtlichkeit halber eingezeichnete räumliche Trennung muß dabei zwingend im gesamten Strahlengang vorhanden sein, gegebenenfalls können geeignete nicht dichroitische Teiler eingesetzt werden. Der Aufbau kann dann im wesentlichen dem der Figur 7 entsprechen, wobei die Polteiler 301 und 302 durch entsprechend dichroitische Teiler ersetzt sind und der Polarisator 300 gegen eine entsprechende Überlagerungseinheit der spektral verschiedenen-Strahlen asugetauscht ist.

Figur 12 zeigt den Aufbau des Spektrometers S, das die zwei spektral getrennten Strahlen 306.1 und 306.2. nachweist. Wieder ist zur Verdeutlichung ein räumlicher Abstand zwischen den Strahlen 306. 1 und 306.2 eingezeichnet, der nicht gegeben sein muß. Das Beugungsgitter 42 teilt die beiden überlagerten, spektral verschiedenen Strahlen in unterschiedliche Raumwinkel ab, so daß sie auf eigenständige Photo-Detektorarrays 43.1 sowie 43. 2 geleitet werden können. In der Bauweise der Figur 12 ist zur Aufweitung des Strahlengangs zusätzlich noch ein Umlenkspiegel 310 vorgesehen.

Für den Fall, daß die Lichtquellen 1.1 und 1.2 durchgestimmt werden, kann die spektrale Analyse wiederum entfallen und es sind lediglich die spektral unempfindlichen Detektoren 309.1 bzw. 309.2 vonnöten. Hierzu müssen die überlagerten Strahlen 306. 1 und 306.2 allerdings durch geeignete Mittel, die dem Fachmann bekannt sind, räumlich getrennt sein.

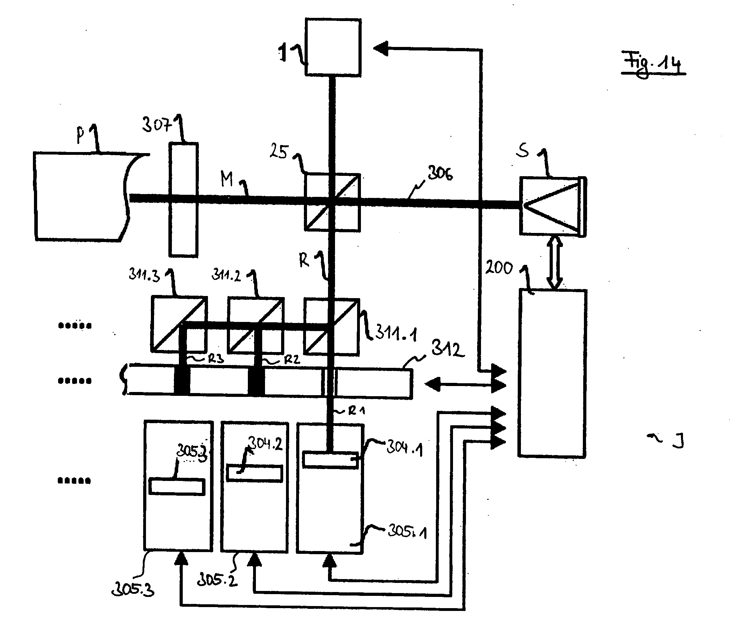

Natürlich kann statt zwei eigenständigen Lichtquellen 1.1 und 1.2 auch eine Lichtquelle verwendet werden, die zwei chromatisch verschiedene Strahlen simultan abgibt. Figur 14 zeigt eine weitere Abwandlung des Spektrometers der Figuren 1-10, wobei nun allerdings keine gleichzeitige Messung mit Überlagerung aus den Referenzstrahlengängen R1, R2 usw. erfolgt, sondern eine sequentielle Überlagerung und Messung. Der Aufbau entspricht im wesentlichen dem der Figur 7, jedoch sind keine polarisationswirksamen Elemente eingesetzt.

Der Referenzstrahlengang R weist statt dessen mehrere Strahlteiler 311 auf, die den Strahlengang in mehrere Referenzstrahlengänge R1, R2, ... aufteilen, die jeweils in entsprechenden Reflektoren 304 enden. Jeder Reflektor 304 ist auf einen Schlitten 305 befestigt. Es sind im Ausführungsbeispiel drei verschiedene Referenzstrahlengänge R1, R2, R3 gebildet. Den Strahlteilern 311.1, 311.2 bzw. 311.3 ist ein Blendenrad 312 nachgeordnet, das ein-Wahlmittel realisiert, welches festlegt, welcher der Referenzstrahlengänge R1, R2, R3 aktiv ist. Die anderen sind abgeschaltet. In der Darstellung der Figur 14 ist der Referenzstrahlengang R1 aktiv, d. h. dessen Referenzstrahl wird am Spiegel 304.1 reflektiert. Durch Umschalten des Blendenrads 312 wird also jeweils ein unterschiedlich langer Referenzstrahlengang aktiv geschaltet, so daß der überlagerte Strahl 306 immer eine Überlagerung aus dem Meßstrahl M mit dem Referenzstrahl aus dem entsprechend aktivierten Referenzstrahlengang gebildet ist. Das Spektrometer S weist dann das entsprechende Signal nach. Die Länge der Referenzstrahlengänge R1, R2, R3 (natürlich kann eine beliebige Anzahl von Referenzstrahlengängen verwendet werden) bewirkt, wie bereits erwähnt, die Tiefenauswahl des Objektbereiches in der Probe P.

Verwendet man für die zuvor oder nachfolgend beschriebenen Interferometer eine Breitbandlichtquette 1 sind das bzw. die Spektrometer S vorzugsweise im sogenannten Czerny-Turner-Aufbau realisiert. Beleuchtet man die Probe P bzw. das Auge A mit mehreren Spots oder mit einer Beleuchtungslinie, was für alle hier beschriebenen Bauweisen möglich ist, sind Spektrometer S mit entsprechenden Detektorarrays ausgestattet, die das spektrale Interferenzmuster auf verschiedenen Detektorarrayzeilen aufnehmen.

Bei einer durchgestimmten Lichtquelle 1 bieten sich anstelle der Spektrometer S, wie bereits erwähnt, Photodioden oder Monochromatoren an, die für den durchgestimmten Spektralbereich der Lichtquelle 1 in der Regel am empfindlichsten sind. Werden hier mehrere Spots oder eine Zeile bzw. Linie in der Probe P beleuchtet, ist der Detektor wiederum mit der entsprechenden Anzahl von Photodioden oder Pixel ausgestattet. Grundsätzlich muß der Photodetektor das Interferenzmuster synchronisiert zur Durchstimmung der Lichtquelle aufzeichnen. Dies erfolgt durch geeignete Steuerung des Computers 200, der eine exemplarische Realisierung eines Steuergeräts ist, das den Betrieb des Interferometers I steuert.

Das Steuergerät ermöglicht insbesondere eine voll automatische zwei- oder drei-dimensionale Bildgewinnung.

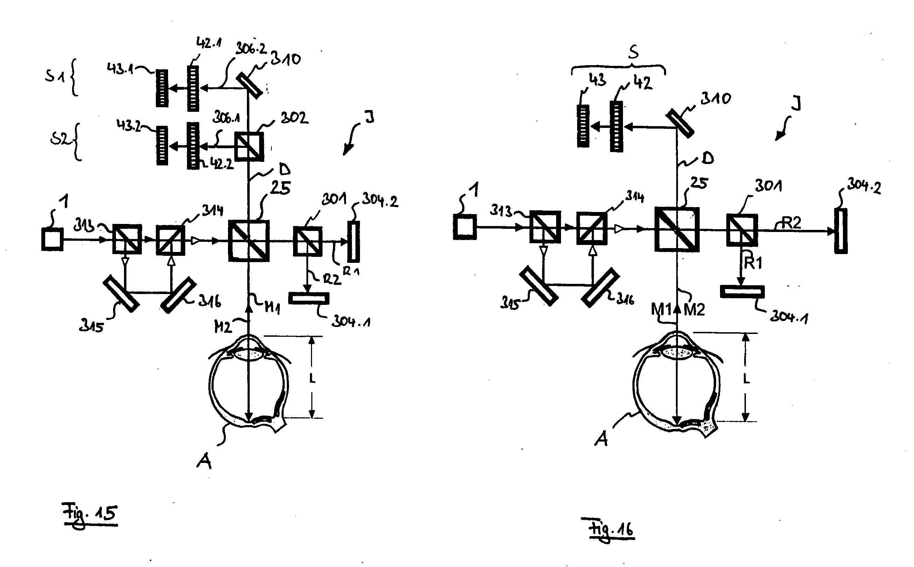

Figur 15 zeigt eine Variante des Spektrometers der Figur 7, wobei das Auge nun mit einem Doppelstrahl beleuchtet wird. Elemente, die bereits in Figur 7 geschildert wurden, werden hier deshalb nicht noch einmal beschrieben. Der Doppelstrahl stellt zwei senkrecht zueinander polarisierte Meßstrahlen M1 und M2 zur Verfügung, wobei diese Strahlen koaxial laufen und gegeneinander versetzt sind. Das Interferometer I der Figur 15 verwendet also zwei Meßstrahlen M1 und M2 und zwei Referenzstrahlen R1 und R2. Damit sind zwei gleichzeitige Messungen einfach möglich, beispielsweise Position der Cornea und Position des Fundus. Die Aufteilung des Doppelstrahls mittels Polteilem 313, 314 und Umlenkspiegeln 315, 316 erlaubt es weiter, eine separate Fokussierung für die beiden Meßstrahlen M1 und M2 vorzunehmen. Dazu muß lediglich ein Fokuselement in den Umlenkweg zwischen den Polteilern 313 und 314 geschaltet werden. Damit kann beispielsweise der Meßstrahl M1 auf die Augenvorderfläche fokussiert werden, wohingegen der Meßstrahl M2 parallel und damit durch die Augenlinse im Fundus fokussiert einfällt. Die Augenlänge L erhält man als Summe aus dem Versatz (= der Wegdifferenz) der zwei Meßstrahlen M1 und M2 aufgrund der Strahlaufspaltung durch die Strahlteiler 313 und 314, plus die Wegdifferenz der Referenzstrahlen R1 und R2, plus eine noch verbleibende optische Wegdifferenz die sich aus der Differenz der Positionen der Signalpeaks der zwei Fouriertransformierten der zwei K-Spektren ergibt.

Figur 16 zeigt eine Abwandlung des Interferometers I der Figur 15,- bei dem nun-für die zwei Meßstrahlen M nur ein Spektrometer S vorgesehen ist. Dazu ist die Summe der Wegdifferenzen zwischen den beiden Meßstrahlen M1 und M2 und den zugehörigen Referenzstrahlen R1 und R2 geeignet auf Werte kleiner als die Meßfeldtiefe z eingestellt. Die Augenlänge ergibt sich dann, wie für Figur 15 schon beschrieben; die Differenz der Positionen der zwei Signalpeaks der zwei Fouriertransformierten wird am Computer-Monitor abgelesen Diese Einstellung kann dadurch bewirkt werden, daß man die Wegdifferenzen durch einen Verstellmechanismus, beispielsweise einen Verschiebemechanismus der zuvor für andere Interferometer geschilderten Art, entweder am jeweiligen Meßstrahl oder Referenzstrahl einstellt.

Eine Alternative besteht darin, einen Meßstrahl als Referenzstrahl zu verwenden und die optische Weglänge der Strahlen geeignet so einzustellen, daß wiederum zwei Meßsignale, also die Autokorrelationsfunktion (AKF) des Referenzstrahles und das Interferenzsignal, am Computer-Monitor dargestellt werden. Nun ergibt sich die Augenlänge als Summe aus Wegdifferenz zwischen den beiden Strahlen plus am Monitor abgelesene Positionsdifferenz der zwei Fourier-Transformierten. Die Vorrichtung ist damit auf Bewegungen des Meßobjektes unempfindlich.

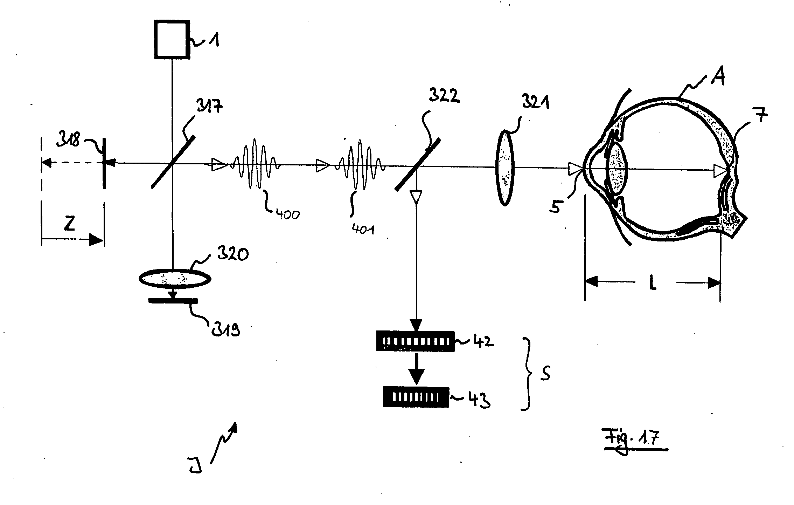

Für die Messung der Augenlänge ist es vorteilhaft, das Interferenzmuster hinsichtlich der Weglängendifferenz auf die Corneavorderfläche zu beziehen: Dies kann man dadurch erreichen, daß der Referenzstrahlengang einen Reflex von der Cornea ausnutzt, also das Referenzstrahlbündel an der Cornea reflektiert wird. Ein entsprechender Aufbau ist in Figur 17 dargestellt. Bauteile die von den zuvor geschilderten Interferometern I unverändert oder funktionsgleich übernommen wurden, sind hier wieder mit denselben Bezugszeichen versehen und werden nicht noch einmal erläutert.

Der Strahlengang der Figur 17 ist der eines Kurzkohärenz-Interferometers I, das einen Doppelstrahl aus zwei Strahlen 400, 401, die koaxial zueinander sind und gegeneinander versetzt sind, auf das Auge richtet. Der vorauseilende Strahl 401 wirkt als Referenzstrahl, der an der Corneavorderfläche reflektiert wird. Die Weglängendifferenz zwischen den Strahlen 400 und 401 wird dabei über Spiegel 318 und 319 eingestellt, die über einen Strahlteiler 317 beaufschlagt werden. Die Weglängendifferenz entspricht im wesentlichen der zu erwartenden Augenlänge L. Die voreingestellte Weglängendifferenz sorgt dafür, daß der Referenzstrahl 401 zusammen mit dem am Fundus reflektierten Meßstrahl 400 interferiert, so daß das einem Strahlteiler 322 nachgeordnete Spektrometer ein entsprechendes Interferenzsignal aufzeichnet. Eine Fourier-Auswertung des Signals zeigt dann zum einen die Autokorrelationsfunktion des Referenzstrahls 400, der mit sich selbst interferiert, und zum anderen einen entsprechenden Abstand für die Überlagerung des Referenzstrahles 401 mit dem Meßstrahl 400. Zusätzlich ist noch die Weglängendifferenz Z, die extern eingestellt wurde, zu berücksichtigen. Die Länge L des Auges A ergibt sich also aus der Summe aus dem Meßergebnis der optischen Messung plus der mechanisch eingestellten Weglängendifferenz zwischen 400 und 401. Die Voreinstellung ermöglicht die FD-Analyse mit einfachen Mitteln, bei vergleichsweise geringem Meßbereich.

Das Interferometer der Figur 17 ist ein Beispiel, bei dem der Referenzstrahl an der Probe reflektiert ist. Dadurch ist eine automatische Relativmessung erreicht und der Abstand wird nicht durch Differenz zweier absoluter Meßpunkte ermittelt. Ortsvarianten der Probe sind dadurch zu vernachlässigen.

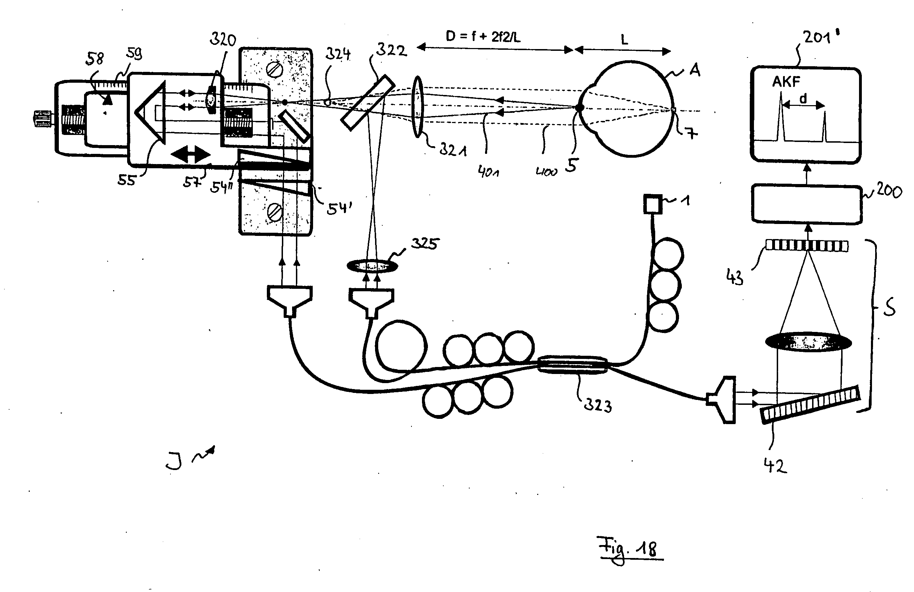

Figur 18 zeigt eine faseroptische Ausbildung des Interferometers I der Figur 17. Wiederum sind unverändert oder funktionsgleich übernommene Bauteile mit denselben Bezugszeichen versehen. Die Bildung der Strahlen 400, 401 erfolgt nun unter Verwendung eines Kopplers 323, der zwei Fasern speist, die den Referenzstrahl 401 und den Meßstrahl 400 abgeben. Der Referenzstrahl 400 ist hinsichtlich der Weglänge durch einen Verschiebemechanismus einstellbar, wie er zuvor bereits beispielsweise anhand der Figur 1 erläutert wurde. Zusätzlich ist auf dem Verschiebemechanismus aber noch die Optik 320 angeordnet, so daß sich mit der Verschiebung automatisch auch eine geeignete Fokussierung auf die Cornea 5 ergibt. Weiter sind die Dispersions-Kompensationsprismen 54' und 54" in den Verschiebemechanismus derart eingebunden, daß eine Längenänderung des Lichtweges automatisch auch eine entsprechende angepaßte, d. h. dynamische Dispersions-Kompensation zur Folge hat. Ansonsten entspricht der Aufbau funktionell dem der Figur 17. '

Zusätzlich ist zur Verdeutlichung noch das nach der Auswertung durch den Computer 200 dargestellte Monitor-Bild 201' gezeigt. Auch ist Figur 18 zu entnehmen, daß der Meßstrahl M1, der zugleich auch als Referenzstrahl R wirkt, auf die Corneavorderfläche fokussiert ist. Es ist damit ein dynamischer Fokus erreicht, der an verschiedene Augenlängen angepaßt werden kann. Durch die verstellbare Optik 325 ist der Meßstrahl M2 so kollimiert, daß die optische Wirkung des Auges A den Meßstrahl M2 am Fundus 7 fokussiert.

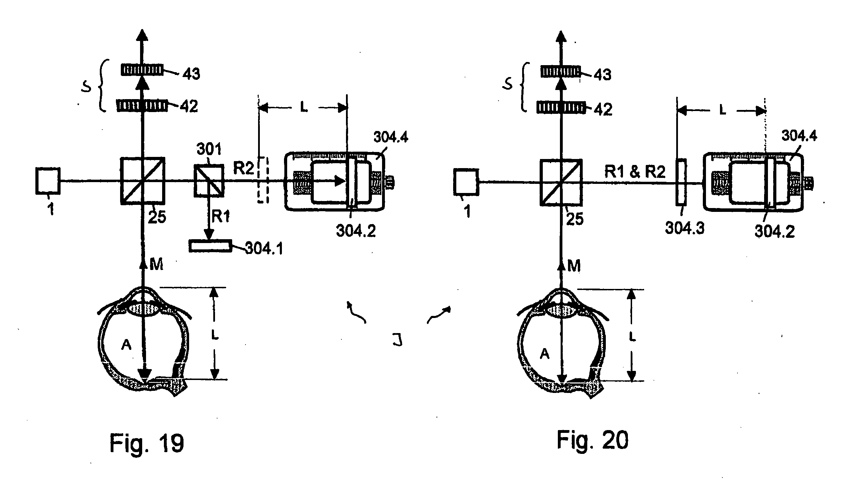

Bei verminderten Ansprüchen an die Signalqualität kann man die Strahlseparations-Vorrichtungen vereinfachen. Dann hat man mehrere Meßsignale gleichzeitig am Array und in der Fouriertransformierten. Entsprechende Strahlengänge sind in den Figuren 19 und 20 abgebildet. Es handelt sich um Vereinfachungen der Strahlengänge der Bauweise gemäß Figur 16. Der Referenzspiegel 304.2 ist nun in beiden Anordnungen auf einem Verschiebetisch 304.4 montiert. Durch Bewegen dieses. Verschiebetisches kann man an der synchronen Bewegung des zugehörigen Signalpeaks der Fouriertransformierten erkennen, zu welcher Probenfläche er gehört.

In der Figur 20 ist der Referenzspiegel 304.1 durch einen teildurchlässigen Spiegel 304.3 ersetzt. Es können zwischen dem Strahlteiler 25 und dem Referenzspiegel 304.2 noch weitere teildurchlässige Referenzspiegel angeordnet werden. So können gleichzeitig zwei oder mehrere Referenzstrahlen im Referenzstrahlengang benutzt werden und mehrere in der Tiefe beabstandete Meßbereiche erfaßt werden. Diese weiteren Referenzspiegel können auf Verschiebetischen montiert werden, um die Meßflexibilität zu erhöhen.

Die Bauweise der Figur 20 kann noch weiter abgewandelt werden. Dann weist das Spektrometer S ein Detektor-Array 43 mit mindestens 7000 Pixeln auf, z. B. 8000. Es wird dann mit nur einem Meßstrahl gearbeitet, und der teildurchlässige Spiegel 304.3 entfällt. Dadurch liegt im Interferometer 1 nur noch ein Referenzstrahlengang R vor. Die Augenlängenmessung erfolgt durch das pixelreiche Array 43 in Kombination mit geeigneter spektraler Auffächerung durch das Beugungsgitter 43 in einer Messung.

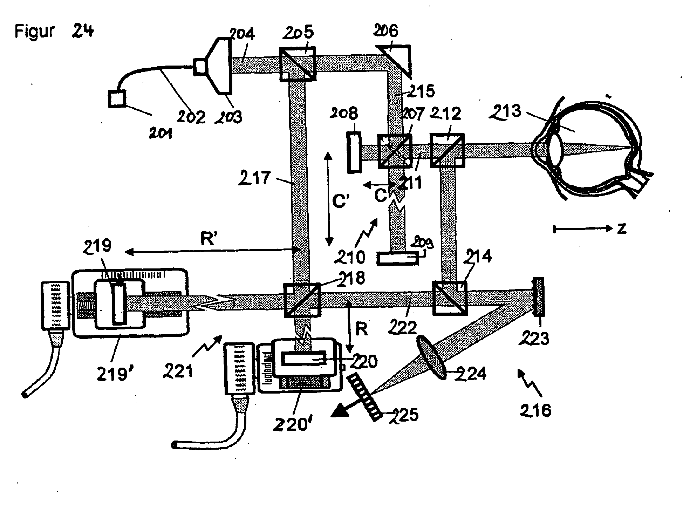

In Figur 24 bezeichnet 201 eine in der Kurzkohärenz-Interferometrie übliche Lichtquelle, beispielsweise eine Superlumineszenz-Diode. Eine Lichtleitfaser 202 leitet die Strahlung zu einem Kollimator 203, der das aus der Lichtleitfaser emittierte Lichtbündel 204 kollimiert und durch einen Strahlteiler 205 hindurch als Meßstrahl 215 über einen Umlenkspiegel 206 zu einem Strahlteiler 207 reflektiert. Der Strahlteiler 207 bildet mit den Spiegeln 208 und 209 ein Michelson-Interferometer 210, welches einen Doppelstrahl 211 erzeugt, der durch einen Strahlteiler 212 hindurch auf ein Auge 213 gerichtet wird. Die von den Grenzflächen des Auges 213 reflektierten und zurück gestreuten Lichtwellen werden von einem Strahlteiler 212 zu einem Strahlteiler 214 und von diesem zu einem Spektrometer 216 gespiegelt.

Das vom Strahlteiler 205 gespiegelte Lichtbündel 217 trifft auf einen Strahlteiler 218, der mit Spiegeln 219 und 220 ein weiteres Michelson-Interferometer 221 bildet, welches einen Referenz-Doppelstrahl 222 erzeugt, der durch den Strahlteiler 214 hindurch ebenfalls zum Spektrometer 216 gespiegelt wird. Das Spektrometer 216 besteht aus einem Reflexionsgitter 223, einer Spektrometeroptik 224 und einem linearen Detektor-Array 225. Alternativ kann auch ein Spektrometer mit einem Transmissionsgitter oder einem anderen dispersiven Element benutzt werden.

Die Messung intraokulärer Distanzen erfolgt so, daß die zwei Referenzspiegel 219 und 220, beispielsweise mit Hilfe von Schrittmotor- oder Piezomotor-gesteuerten Positionierem 219' und 220', so verschoben werden, daß die Signalspitzen jener Grenzflächen im Meßfeld sichtbar werden, die die zu messende Distanz definieren (vgl. Figur 21). Für die in Figur 21 angedeutete Messung der Augenlänge L ist beispielsweise

$$L = S + (R' - R) - (C' - C)$$

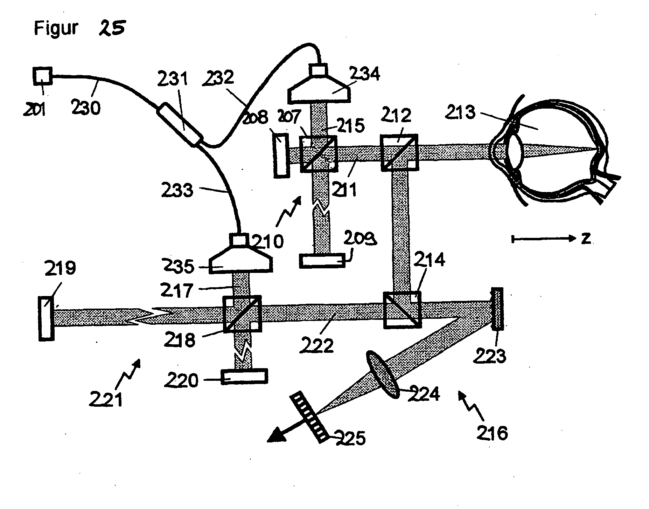

In Figur 25 ist ein Teil des Interferometers der Figur 24 faseroptisch ausgeführt (strukturell oder funktionell unveränderte Bauteile sind in den Figuren generell mit den gleichen Bezugszeichen versehen). Hier wird das von der Kurzkohärenz-Lichtquelle 201 emittierte Licht von einer Lichtleitfaser 230 zu einem Koppler 231 geführt und von diesem in Meßstrahl und Referenzstrahl geteilt, die durch Fasern 232 und 233 und Kollimatoren 234 und 235 den Interferometern 210 und 221 zugeführt werden. Der restliche Strahlengang entspricht dem der Figur 24.

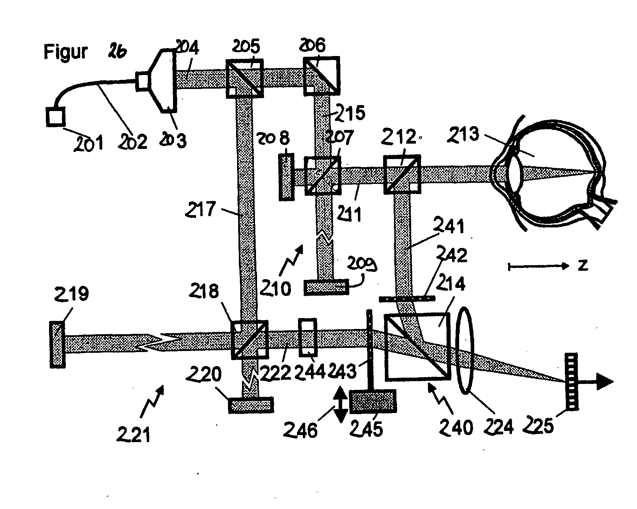

In Figur 26 ist das Kurzkohärenz-Interferometer mit einem Doppel-Spektrometer 240 ausgerüstet. Es besteht aus zwei gleichen Beugungsgittern 242 und 243, einem Strahlteiler 214, der hier die an den Gittern 242 und 243 gebeugten Strahlen zusammenführt, und dem linearen Detektor-Array 225. Die vom Auge 213 reflektierten und zurückgestreuten Lichtwellen 241 werden von dem Beugungsgitter 242 gebeugt, und der Doppel-Referenzstrahl 222 wird von dem Beugungsgitter 243 gebeugt; beide Beugungsbilder überlagern ihr Beugungsbild auf dem linearen Detektor-Array 225. Man muß die zwei Beugungsgitter 242 und 243 durch Beobachtung von der Array-Seite her so einjustieren, daß sie kongruent sind. Dann arbeitet das in der Figur 6 dargestellte Kurzhohärenz-Interferometer genauso, wie das der Figuren 4 und 5. Das Detektor-Array 225 registriert die reelle Intensität $I(k)$ des (komplexen) Frequenzspektrums $I(k)$ der Strahlung am Interferometer-Ausgang.

Das Beugungsgitter 243 des Doppel-Spektrometers 240 ist mit einem piezoelektrischen Aktuator 245 verbunden, der es in der Gitterebene normal zu den Gitterlinien um 1/4 der Gitterkonstante verschieben kann (Doppelpfeil 246). Damit erhalten die am Gitter gebeugten Wellen eine Phasenverschiebung von $\pi/2$. In dieser Position registriert daher das Detektor-Array 225 die zur reellen Intensität $I(k)$ zugehörige Quadraturkomponente, mit der man nach obiger Gleichung 3 das komplexe Frequenzspektrum $\hat{i}(k)$ der Strahlung am Interferometer-Ausgang erhält.

Analog kann auch das Gitter 242 um 1/4 der Gitterkonstante verschoben werden. Alternativ kann eine Phasenverschiebung der Referenzstrahlen auch mittels eines elektrooptischen Phasenmodulators 244 im Doppel-Referenzstrahl 222 erfolgen. Dieser kann auch im Doppel-Meßstrahl 241 angeordnet werden. Während jedoch die Gitter-Methode von der Wellenlänge unabhängig wirkt, ist dies bei der Phasenmodulator-Methode nicht der Fall; letztere Methodekann bei Wellenlängen-Bandbreiten bis zu einigen 10 nm verwendet werden.

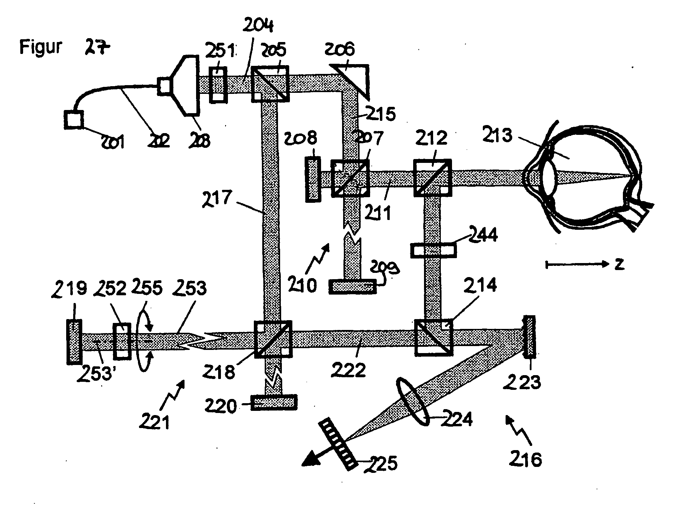

Figur 27 stellt eine Anordnung zur Augen-Teilstreckenmessung mittels Fourier-Domain-Interferometrie dar, die zur Achslängenmessung selektiv das retinale Pigmentepithel (RPE) benutzt und das von den anderen retinalen Schichten zurück gestreute Licht polarisationsoptisch diskriminiert. Hierzu befindet sich in dem vom Kollimator 203 abgestrahlten Lichtbündel 204 ein Linear-Polarisator 251. Dieser stellt einen definierten Polarisationszustand des Lichtbündels 204, welches das Kurzkohärenz-Interferometer beleuchtet, sicher. Ferner befindet sich in jenem Referenz-Teilstrahl 253, der mit dem vom Fundus des Auges 213 reflektierten Licht interferiert, eine $\lambda/4$-Platte 252 unter 45° zur Polarisationsebene. Hierdurch entsteht zirkular polarisiertes Licht, das durch die Reflexion am Referenzspiegel 219 seine Drehrichtung umkehrt. Beim nochmaligen Durchlaufen der $\lambda/4$-Platte 252 entsteht wieder linear polarisiertes Licht, nunmehr jedoch mit um 90° gedrehter Polarisationsebene, also normal zur ursprünglichen Polarisationsebene. Bei normal zur ursprünglichen Richtung polarisiertem Licht führt lediglich das vom RPE reflektierte Licht, welches in seiner Polarisationsrichtung verändert ist, zu Interferenzen. So erhält man die Distanz zwischen Cornea-Vorderfläche und RPE, also eine Augen-Achslängenmessung auf Basis des RPE.

Durch Drehen der $\lambda/4$-Platte (Doppelpfeil 255) um eine Achse 253' parallel zur Achse des Strahls 253 kann der Anteil des parallel zur ursprünglichen Polarisationsebene polarisierten Lichts verändert werden. Hierdurch wird die Stärke der Interferenzen mit dem vom Fundus reflektierten Licht eingestellt, und neben dem RPE können auch andere Grenzschichten der Retina sichtbar gemacht werden.

Analog hierzu kann die Polarisationsebene des Strahls 253 mit Hilfe einer um die Strahlachse 253' drehbaren $\lambda/2$-Platte anstatt der Platte 252 gedreht werden. Dies verändert die Stärke der Interferenzen mit dem vom Fundus reflektierten Licht.

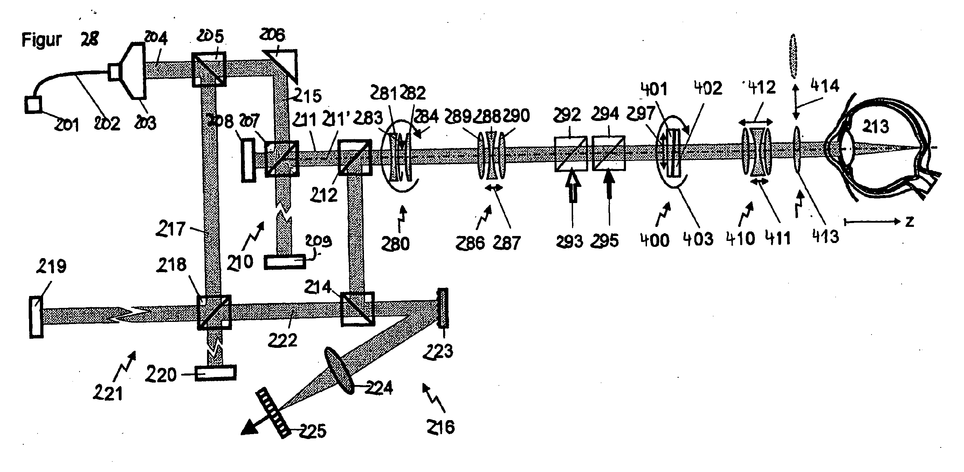

Weitere Anpassungen an verschiedene Meßumstände sind durch die in Figur 28 angeführten Hilfseinrichtungen möglich, die prinzipiell im Meßstrahlengang und insbesondere darin im Doppel-Meßstrahl 211 angeordnet oder darin eingespiegelt werden können:

Zur Kompensation des Astigmatismus der Probandenaugen kann eine Linsengruppe 280 aus zwei Zylinderlinsen 281 und 282 entgegengesetzter Brechkraft benutzt werden. Durch Verdrehen ihrer Zylinderachsen um die Achse des Meßstrahls (z. B. 211) relativ zueinander (Doppelpfeil 283) können unterschiedliche zylindrische Brechkräfte der Linsengruppe realisiert werden. Durch Drehen der gesamten Linsengruppe (Doppelpfeil 284) um die Achse-211' des Meßstrahls kann die Orientierung der Zylinderachse der Gruppe verändert werden. Mit dieser Hilfseinrichtung kann man im Meßstrahl einen dem Probandenauge entgegengesetzten Astigmatismus erzeugen und den Probanden-Astigmatismus kompensieren.

Eine Zoom-Optik 286 (beispielsweise mit einer verschiebbaren - Doppelpfeil 287 - Zerstreungslinse 288 zwischen zwei Sammellinsen 289 und 290) kann dazu verwendet werden, eine Ametropie des Probandenauges zu kompensieren. Diese Zoom-Optik hat eine Mittelstellung mit Brechkraft Null und kann positive als auch negative Brechkräfte erzeugen. Mit Hilfe eines Strahlteilers 292 kann eine Refraktometrie-Vorrichtung, beispielsweise ein Hartinger Koinzidenz-Refraktometer (offener Pfeil 293) eingespiegelt werden. Analog kann mit Hilfe eines Strahlteilers 294 ein Fixierlicht (Pfeil 295) eingespiegelt werden, um die Sehachse des Probandenauges 213 festzulegen. Das Fixierlicht kann farblich unterschiedlich (z. B. grün) sein. Auch kann es durch blinken hervorgehoben werden. Zur Festlegung bzw. Variation der Blickrichtung kann positionsverstellbares Fixierlicht verwendet werden.

Die Gruppe 300 besteht aus zwei Keilen 301 und 302, die relativ zueinander verschiebbar sind (Doppelpfeil 297). Hierdurch kommt es zu einer Deviation des Meßstrahls (z. B. 211 deren Größe durch den Betrag der relativen Prismenverschiebung und deren Richtung durch Drehen (Doppelpfeil 103) der Prismengruppe um die Achse 211' des Doppel-Meßstrahls 211 verändert werden kann. So kann die optische Achse des fixierten Auges parallel zur Strahlachse 211' des Meßstrahls eingestellt werden, um optimale Lichtreflexe aus dem Auge zu erhalten.

Eine Zoom-Optik 310 dient für Messungen an der Vorderkammer des Probandenauges. Sie ist so ausgelegt, daß ihre Brechkraft von Null bis zu mehreren Dioptrien einstellbar ist. Die durch diese Optik erzeugte Fokussierung des Meßstrahls (z. B. 211) in oder nahe an die Vorderkammer erhöht die Signalstärke der aus der Vorderkammer reflektierten Lichtanteile. Zustarke Fokussierung jedoch erfordert sehr sorgfältige Einstellung und Beibehaltung der transversalen Position des Auges, was schwierig ist. Man wird daher das Optimum bei einer geringeren Fokussierung finden. Dieses muß empirisch über entsprechende Einstellungen der Zoom-Optik 310 gefunden werden. Hierzu kann diese Zoom-Optik neben einer Brechkraftverstellung (Doppelpfeil 311) auch eine Verstellmöglichkeit ihrer Position in Richtung der optischen Achse des Meßstrahls besitzen (Doppelpfeil 312). Auch kann man anstelle der Zoom-Optik Optiken 313 entsprechender fixer Brechkraft in den Strahlengang einschwenken (Doppelpfeil 314), und ebenfalls in Richtung der optischen Achse 211' verstellbar anordnen.

Die oben angeführten Hilfseinrichtungen können alle zusammen im Meßstrahlengang (z. B. den Doppel-Meßstrahl 211) angeordnet werden. Alternativ können natürlich auch einzelne oder einige dieser Einrichtungen angeordnet werden. Die Gruppe 300 erfordert jedoch meist eine separat Fixierung des Auges 213 und sollte daher nur gemeinsam mit der Augenfixierung mit den Komponenten 294 und 295 verwendet werden. Da der Proband bei den hier üblicherweise benutzten Wellenlängen den Meßstrahl (z. B. 211) rot sieht, ist, wie erwähnt, für das eingespiegelte Licht eine andere Farbe, beispielsweise grün, vorteilhaft.

Es sei noch erwähnt, daß neben der Konfiguration eines FDI nach Figur 24 auch die anderen Konfigurationen analog zu Figur 25 faseroptisch modifiziert werden können.

Das erfindungsgemäßen Verfahren wurden im obigen Text anhand der Messung der Achslänge des Auges beschrieben. Es sei jedoch hier auch explizit darauf hingewiesen, daß diese Verfahren auch zur Messung anderer intraokulärer Distanzen, wie der Corneadicke, der Vorderkammertiefe und der Linsendicke eingesetzt werden können. Hierzu müssen lediglich die Referenzspiegel 219 und 220 in solche Positionen verschoben werden, daß die entsprechenden Reflexe in den zwei Meßfenstem sichtbar werden. Die Längenmessung erfolgt analog wie oben im Zusammenhang mit Gleichung 4 beschrieben.

## Patentansprüche

1. Vorrichtung zur interferometrischen Messung einer Probe (P), insbesondere des Auges (A), mit einer Kurzkohärenz-Interferometeranordnung (I), welche einen Meßstrahlengang, durch den ein Meßstrahl auf die Probe (P) fällt, und einen ersten Referenzstrahlengang (R) aufweist, durch den ein Referenzstrahl läuft, der mit dem Meßstrahl überlagert und zur Interferenz gebracht wird, **dadurch gekennzeichnet, daß**
   die Interferometeranordnung (I) mindestens einen zweiten Referenzstrahlengang (R2) aufweist, dessen optischen Weglänge sich von der des ersten Referenzstrahlenganges (R1) unterscheidet, wobei die Weglängendifferenz gemäß einem Abstand zweier in Tiefenrichtung der Probe (P) beabstandeter Probenbereiche (5, 6, 7) gewählt ist und wobei eine Steuereinrichtung (200) aus den detektierten überlagerten Strahlen mittels Fourier-Spektralanalyse unter Berücksichtigung der Weglängendifferenz der Referenzstrahlengänge (R1, R2) den Abstand der Probenbereiche (5, 6, 7) ermittelt.

2. Vorrichtung nach Anspruch 1, **dadurch gekennzeichnet, daß** die Interferometeranordnung (I) eine Überlagerungseinrichtung (25, 322) aufweist, die die Referenzstrahlen (53, 63, 73) aus den Referenzstrahlengängen (R1, R2) getrennt mit dem Meßstrahl (M) aus dem Meßstrahlengang überlagert und die derart überlagerten Strahlen dann an eine Detektoreinrichtung (S) zum Nachweis weiterleitet.

3. Vorrichtung nach Anspruch 2, **dadurch gekennzeichnet, daß** die Überlagerungseinrichtung einen Umschaltmechanismus (322) zum Umschalten zwischen den zwei Referenzstrahlengängen (R1, R2) aufweist, so daß die Überlagerung für die zwei Referenzstrahlengänge (R1, R2) sequentiell erfolgt.

4. Vorrichtung nach Anspruch 2, **dadurch gekennzeichnet, daß** die Überlagerungseinrichtung (25) zur getrennten Überlagerung eine Polarisationstrennung einsetzt, so daß die Überlagerung und das Weiterleiten zur Detektoreinrichtung (S) für die zwei Referenzstrahlengänge (R1, R2) nach Polarisation getrennt und gleichzeitig erfolgt.

5. Vorrichtung nach Anspruch 2, **dadurch gekennzeichnet, daß** die Überlagerungseinrichtung (25) zur getrennten Überlagerung eine dichroitische Trennung einsetzt, so daß die Überlagerung und das Weiterleiten zur Detektoreinrichtung (S) für die zwei Referenzstrahlengänge (R1, R2) spektral getrennt und gleichzeitig erfolgt.

6. Vorrichtung nach Anspruch 2, **dadurch gekennzeichnet, daß** die Überlagerungseinrichtung (25) zur getrennten Überlagerung den Meßstrahl (M) in Meßstrahlbündel räumlich auftrennt und die aufgetrennten Meßstrahlbündel jeweils mit einem der Referenzstrahlen (53, 63, 73) aus einem der Referenzstrahlengänge (R1. R2, R3) überlagert, wobei zur räumlichen Auftrennung insbesondere eine Pupillenteilung eingesetzt ist.

7. Vorrichtung nach einem der obigen Ansprüche, **dadurch gekennzeichnet, daß** mindestens ein Spektrometer (S) vorgesehen ist, das die überlagerten Strahlen spektral analysiert und Meßsignale an die Steuereinrichtung (200) leitet.

8. Vorrichtung nach einem der Ansprüche 1 bis 6, **dadurch gekennzeichnet, daß** eine die Interferometeranordnung speisende, spektral durchstimmbare Strahlungsquelle (1) und eine spektral nicht-auflösende Detektoreinrichtung (S) zur Messung vorgesehen sind, wobei die Detektoreinrichtung (S) Meßsignale an die Steuereinrichtung (200) leitet, die unter Berücksichtigung der Durchstimmung der Strahlungsquelle (1) die Fourier-Spektralanalyse durchführt.

9. Vorrichtung nach einem der obigen Ansprüche, **dadurch gekennzeichnet, daß** im Meßstrahlengang mehrere coaxiale und axiale gegeneinander versetzte Meßstrahlen (M1, M2; 211) auf die Probe fallen.

10. Vorrichtung nach Anspruch 9, **dadurch gekennzeichnet, daß** für jeden Meßstrahl genau ein Referenzstrahl vorgesehen ist.

11. Verfahren zur kurzkohärenz-interferometrischen Messung einer Probe, insbesondere des Auges, wobei durch einen Meßstrahlengang ein Meßstrahl auf die Probe gerichtet wird und mit einem Referenzstrahl, der einen ersten Referenzstrahlengang durchläuft, überlagert und zur Interferenz gebracht wird, **dadurch gekennzeichnet, daß** mindestens ein zweiter Referenzstrahlengang vorgesehen wird, dessen optische Weglänge sich von der des ersten Referenzstrahlenganges unterscheidet, wobei die Weglängendifferenz gemäß einem Abstand zweier in Tiefenrichtung der Probe beabstandeter Probenbereiche gewählt wird und die überlagerte Strahlung detektiert und daraus mittels Fourier-Spektralanalyse unter Berücksichtigung der Weglängendifferenz der Referenzstrahlengänge der Abstand der Probenbereiche ermittelt wird.

12. Verfahren nach Anspruch 11, **dadurch gekennzeichnet, daß** die Strahlen aus den zwei Referenzstrahlengängen getrennt mit dem Meßstrahl aus dem ersten Meßstrahlengang überlagert, die derart überlagerten Strahlen detektiert und den beabstandeten Probenbereichen zugeordnete Meßsignale erzeugt werden.

13. Verfahren nach Anspruch 12, **dadurch gekennzeichnet, daß** zwischen den zwei Referenzstrahlengängen sequentiell umgeschaltet wird, so daß der Meßstrahl sequentiell mit Strahlung aus dem ersten und dem zweiten Referenzstrahlengang überlagert wird und die überlagerten Strahlen sequentiell detektiert werden.

14. Verfahren nach Anspruch 12, **dadurch gekennzeichnet, daß** zur getrennten Überlagerung eine Polarisationstrennung einsetzt wird, so daß die getrennte Überlagerung und Detektion für die zwei Referenzstrahlengänge nach Polarisation getrennt und gleichzeitig erfolgt.

15. Verfahren nach Anspruch 12, **dadurch gekennzeichnet, daß** zur getrennten Überlagerung eine dichroitische Trennung einsetzt wird, so daß die getrennte Überlagerung und Detektion für die zwei Referenzstrahlengänge spektral getrennt und gleichzeitig erfolgt.

16. Verfahren nach Anspruch 12, **dadurch gekennzeichnet, daß** zur getrennten Überlagerung eine räumliche Auftrennung des Meßstrahls in Meßstrahlbündel erfolgt und die aufgetrennten Meßstrahlbündel jeweils mit einem der Referenzstrahlen überlagert werden, wobei zur räumlichen Auftrennung insbesondere eine Pupillenteilung eingesetzt wird.

17. Verfahren nach einem der obigen Verfahrensansprüche, **dadurch gekennzeichnet, daß** die überlagerten Strahlen spektral selektiv detektiert werden.

18. Verfahren nach einem der Ansprüche 10 bis 16, **dadurch gekennzeichnet, daß** die Strahlung spektral durchgestimmt und die zur Überlagerung gebrachten Strahlen spektral nichtauflösend detektiert werden.

## Claims

1. An apparatus for interferometric measurement of a sample (P), in particular of the eye (A), said apparatus comprising a short-coherence interferometer arrangement (I), which comprises a measurement beam path through which a measurement beam is incident on the sample (P), and a first reference beam path (R), through which a reference beam passes that has superimposed upon it and is made to interfere with the measurement beam, **characterized in that** the interferometer arrangement (I) comprises at least one second reference beam path (R2), whose optical path length differs from that of the first reference beam path (R1), the path length difference being selected according to a distance of two sample regions (5, 6, 7), which are spaced apart in depth direction of the sample (P), and a control device (200) determining from the detected, superimposed beams the distance between the sample regions (5, 6, 7) by a Fourier spectral analysis in consideration of the path length difference of the reference beam paths (R1, R2).

2. The apparatus as claimed in claim 1, **characterized in that** the interferometer arrangement (I) comprises a superimposing device (25, 322), which separately superimposes the measurement beam (M) coming from the measurement beam path upon each of the reference beams (53, 63, 73) coming from the reference beam paths and then transmits the thus superimposed beams to a detector device (S) for detection.

3. The apparatus as claimed in claim 2, **characterized in that** the superimposing device comprises a switching mechanism (322) for switching between the two reference beam paths (R1, R2), so that superposition is effected sequentially for the two reference beam paths (R1, R2).

4. The apparatus as claimed in claim 2, **characterized in that** the superimposing device (25) uses polarization separation for separate superposition, so that superposition and transmission to the detector device (S) for the two reference beam paths (R1, R2) are effected separately, according to polarization, and simultaneously.

5. The apparatus as claimed in claim 2, **characterized in that** the superimposing device (25) uses dichroic separation for separate superposition, so that superposition and transmission to the detector device (S) for the two reference beam paths (R1, R2) are effected in a spectrally separated and simultaneous manner.

6. The apparatus as claimed in claim 2, **characterized in that** the superimposing device (25) spatially separates the measurement beam (M) into measurement ray bundles for separate superposition and superimposes each separated measurement ray bundle upon one of the reference beams (53, 63, 73) coming from one of the reference beam paths (R1, R2, R3), wherein in particular said spatial separation uses a pupil partition.

7. The apparatus as claimed in any one of the above claims, **characterized in that** at least one spectrometer (S) is provided which spectrally analyzes the superimposed beams and transmits measurement signals to the control device (200).

8. The apparatus as claimed in any one of claims 1 to 6, **characterized in that** a spectrally sweepable source of radiation (1), which feeds the interferometer, and a spectrally non-resolving detector device (S) are provided for measurement, said detector device transmitting measurement signals to the control device (200) which carries out Fourier spectral analysis in consideration of the spectral sweep of the source of radiation (1).

9. The apparatus as claimed in any one of the above claims, **characterized in that** several coaxial measurement beams (M1, M2; 211) with a mutual axial offset in the measurement beam path are incident on the sample.

10. The apparatus as claimed in claim 9, **characterized in that** exactly one reference beam is provided for each measurement beam.

11. A method for short-coherence interferometric measurement of a sample, in particular of the eye, wherein a measurement beam is directed onto the sample through a measurement beam path and is superimposed upon and made to interfere with a first reference beam passing through a first reference beam path, **characterized in that** at least one second reference beam path is provided, whose optical path length differs from that of the first reference beam path, wherein the path length difference is selected according to a distance of two sample regions, which are spaced apart in depth direction of the sample, and the superimposed radiation is detected and used to determine the distance between the sample regions by means of Fourier spectral analysis in consideration of the path length difference of the reference beam paths.

12. The method as claimed in claim 11, **characterized in that** the beams from the two reference beam paths have the measurement beam coming from the measurement beam path separately superimposed upon them, the beams thus superimposed are detected, and measurement signals assigned to the spaced-apart sample regions are generated.

13. The method as claimed in claim 12, **characterized in that** sequential switching between the two reference beam paths is effected so that the measurement beam has radiation from the first and second reference beam paths superimposed upon it in sequence and the superimposed beams are sequentially detected.

14. The method as claimed in claim 12, **characterized in that** polarization separation is used for separate superposition such that the separate superposition and detection is effected separately, according to polarization, and simultaneously for the two reference beam paths.

15. The method as claimed in claim 12, **characterized in that** a dichroic separation is used for separate superposition such that the separate superposition and detection is effected in a spectrally separated and simultaneous manner for the two reference beam paths.

16. The method as claimed in claim 12, **characterized in that**, for separate superposition, spatial separation of the measurement beam into measurement ray bundles is effected and the separated measurement ray bundles each have one of the reference beams superimposed upon them, wherein in particular spatial separation is effected using pupil partition.

17. The method as claimed in any one of the above method claims, **characterized in that** the superimposed beams are detected in a spectrally selective manner.

18. The method as claimed in any one of claims 10 to 16, **characterized in that** the radiation is spectrally swept and the superimposed beams are detected in a spectrally non-resolving manner.

**Revendications**

1. Dispositif pour la mesure interférométrique d'un échantillon (P), en particulier de l'oeil (A), comportant un système interférométrique à courte cohérence (I) qui comporte une trajectoire de rayons de mesure par laquelle un rayon de mesure tombe sur l'échantillon (P) et une première trajectoire de rayons de référence (R), par laquelle passe un rayon de référence, qui se superpose au rayon de mesure et qui est amené à interférer, **caractérisé en ce que** le système interférométrique (I) comporte au moins une deuxième trajectoire de rayons de référence (R2) dont la longueur du trajet optique est différente de celle de la première trajectoire de rayons de référence (R1), sachant que la différence de la longueur du trajet optique est choisie en fonction d'une distance entre deux zones (5, 6, 7) de l'échantillon situées dans le sens de la profondeur de l'échantillon (P), et sachant qu'un dispositif de commande (200) détermine, à partir des rayons superposés détectés, la distance entre les zones (5, 6, 7) de l'échantillon au moyen d'une analyse spectrale de Fourier en tenant compte de la différence de longueur des trajectoires des rayons de référence (R1, R2).

2. Dispositif selon la revendication 1, **caractérisé en ce que** le système interférométrique (I) comporte un dispositif de superposition (25, 322), qui superpose séparément les rayons de référence (53, 63, 73), issus des trajectoires des rayons de référence (R1, R2), au rayon de mesure (M) issu de la trajectoire de rayons de mesure et qui transmet ensuite les rayons ainsi superposés vers un dispositif de détection (S) en vue d'une identification.

3. Dispositif selon la revendication 2, **caractérisé en ce que** le dispositif de superposition comporte un mécanisme de permutation (322) pour permuter entre les deux trajectoires des rayons de référence (R1, R2), de telle sorte que la superposition est effectuée séquentiellement pour les deux trajectoires des rayons de référence (R1, R2).

4. Dispositif selon la revendication 2, **caractérisé en ce que** le dispositif de superposition (25) effectue, en vue de la superposition séparée, une séparation de polarisation, de telle sorte que la superposition et le transfert vers le dispositif de détection (S) sont effectués séparément et en même temps pour les deux trajectoires des rayons de référence (R1, R2) après la polarisation.

5. Dispositif selon la revendication 2, **caractérisé en ce que** le dispositif de superposition (25) effectue, en vue de la superposition séparée, une séparation dichroïque, de telle sorte que la superposition et le transfert vers le dispositif de détection (S) sont effectués séparément et en même temps de manière spectrale pour les deux trajectoires des rayons de référence (R1, R2).

6. Dispositif selon la revendication 2, **caractérisé en ce que** le dispositif de superposition (25) effectue, en vue de la superposition séparée, une séparation spatiale du rayon de mesure (M) en faisceaux de rayons de mesure et superpose les faisceaux de rayons de mesure séparés respectivement à l'un des rayons de référence (53, 63, 73), issu de l'une des trajectoires des rayons de référence (R1, R2, R3), sachant qu'en particulier un diviseur de pupille est utilisé pour la séparation spatiale.

7. Dispositif selon l'une quelconque des revendications précédentes, **caractérisé en ce qu'**il est prévu au moins un spectromètre (S) qui analyse de manière spectrale les rayons superposés et transmet les signaux de mesure vers le dispositif de commande (200).

8. Dispositif selon l'une quelconque des revendications 1 à 6, **caractérisé en ce qu'**il est prévu une source de rayonnement (1), ajustable de manière spectrale et alimentant le système interférométrique, et un dispositif de détection (S) sans résolution spectrale, destiné à effectuer la mesure, le dispositif de détection (S) transmettant des signaux de mesure vers le dispositif de commande (200) qui, en tenant compte de l'ajustement de la source de rayonnement (1), effectue l'analyse spectrale de Fourier.

9. Dispositif selon l'une quelconque des revendications précédentes, **caractérisé en ce que**, dans la trajectoire de rayons de mesure, plusieurs rayons de mesure (M1, M2 ; 211) coaxiaux et axiaux, décalés les uns par rapport aux autres, tombent sur l'échantillon.

10. Dispositif selon la revendication 9, **caractérisé en ce qu'**il est prévu exactement un rayon de référence pour chaque rayon de mesure.

11. Procédé pour la mesure interférométrique à courte cohérence d'un échantillon (P), en particulier de l'oeil, un rayon de mesure étant dirigé sur l'échantillon par une trajectoire de rayons de mesure et étant superposé à un rayon de référence, qui passe par une première trajectoire de rayons de référence, et est amené à interférer, **caractérisé en ce que**
il est prévu au moins une deuxième trajectoire de rayons de référence dont la longueur du trajet optique est différente de celle du premier trajectoire de rayons de référence, sachant que la différence de la longueur du trajet optique est choisie en fonction d'une distance entre deux zones de l'échantillon situées dans le sens de la profondeur de l'échantillon, et le rayonnement superposé est détecté et, à partir de là, la distance entre les zones de l'échantillon est déterminée au moyen d'une analyse spectrale de Fourier en tenant compte de la différence de longueur des trajectoires des rayons de référence.

12. Procédé selon la revendication 11, **caractérisé en ce que** les rayons issus des deux trajectoires des rayons de référence sont superposés séparément au rayon de mesure issu de la première trajectoire de rayons de mesure, les rayons ainsi superposés sont détectés et des signaux de mesure, associés aux zones de l'échantillon situées à distance les unes des autres, sont générés.

13. Procédé selon la revendication 12, **caractérisé en ce que** l'on permute séquentiellement entre les deux trajectoires des rayons de référence, de telle sorte que le rayon de mesure est superposé séquentiellement à un rayonnement issu de la première et de la deuxième trajectoire de rayons de référence, et les rayons superposés sont détectés séquentiellement.

14. Procédé selon la revendication 12, **caractérisé en ce qu'**une séparation de polarisation est appliquée pour la superposition séparée, de telle sorte que la superposition séparée et la détection sont effectuées séparément et en même temps pour les deux trajectoires des rayons de référence après la polarisation.

15. Procédé selon la revendication 12, **caractérisé en ce qu'**une séparation dichroïque est appliquée pour la superposition séparée, de telle sorte que la superposition séparée et la détection sont effectuées séparément et en même temps de manière spectrale pour les deux trajectoires des rayons de référence.

16. Procédé selon la revendication 12, **caractérisé en ce qu'**une séparation spatiale du rayon de mesure en faisceaux de rayons de mesure est appliquée pour la superposition séparée et les faisceaux de rayons de mesure séparés sont superposés respectivement à l'un des rayons de référence, sachant qu'en particulier un diviseur de pupille est utilisé pour la séparation spatiale.

17. Procédé selon l'une quelconque des revendications précédentes portant sur le procédé, **caractérisé en ce que** les rayons superposés sont détectés sélectivement de manière spectrale.

**18.** Procédé selon l'une quelconque des revendications 10 à 16, **caractérisé en ce que** le rayonnement est ajusté de manière spectrale et les rayons amenés à interférer sont détectés de manière spectrale sans résolution.

Figur 1

Figur 2

Figur 3

Figur 4

a.

b.

c.

Figur 5

Figur 6

**Fig. 7**

**Fig. 8**

**Fig. 9**

EP 1 959 816 B1

**Fig. 10**

**Fig. 11**

EP 1 959 816 B1

Fig. 14

Fig. 16

Fig. 15

32

Fig. 17

EP 1 959 816 B1

$$D = f + 2f2/L$$

Fig. 18

Fig. 19

Fig. 20

Figur 21

Figur 22

Figur 23

Figur 24

Figur *25*

EP 1 959 816 B1

Figur *26*

204 205 206

201 202 203 215

208 207 212 213

217 211

241

242

210 209 214

219 218

220 222 244 243 240 224 225

221 246 245

z

Figur 27

Figur 28

EP 1 959 816 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 03086180 A
- EP 1602320 A1